# EUROPEAN PATENT SPECIFICATION

(11) **EP 2 298 335 B1**
(45) Date of publication and mention of the grant of the patent: **03.09.2014**
(21) Application number: 10179674.6
(22) Date of filing: 24.05.2005
(51) Int. Cl.: A61K 38/18, A61P 19/02

(54) **Use of OP-1 for treating cartilage defects**
Verwendung von OP-1 zur Behandlung von Knorpeldefekten
Utilisation de OP-1 pour le traitement de défauts du cartilage

(30) Priority: 25.05.2004 US 574423 P
(43) Date of publication of application: 23.03.2011
(62) Divisional of application: 05753766.4
(73) Proprietor: STRYKER CORPORATION, Kalamazoo, MI 49002 (US)
(72) Inventor: Rueger, David, C., Southborough, MA 01772 (US); Kildey, Robyn, Balmain, Sidney, New South Wales 2041 (AU)
(74) Representative: Vossius & Partner

(56) References cited:
- WO-A-00/20021
- WO-A-00/44413
- WO-A-02/067978
- WO-A2-03/063799
- US-A- 5 902 785
- US-A1- 2001 024 823
- US-A1- 2003 175 257
- US-A1- 2003 185 898
- EVANS C H ET AL: "REVIEW OSTEOARTHRITIS GENE THERAPY", GENE THERAPY, MACMILLAN PRESS LTD., BASINGSTOKE, GB, vol. 11, no. 4, February 2004 (2004-02), pages 379-389, XP001180606, ISSN: 0969-7128
- HOLLAND T A ET AL: "Advances in drug delivery for articular cartilage", JOURNAL OF CONTROLLED RELEASE, ELSEVIER SCIENCE PUBLISHERS B.V. AMSTERDAM, NL, vol. 86, no. 1, 9 January 2003 (2003-01-09), pages 1-14, XP004398994, ISSN: 0168-3659

### SEQUENCE LISTING

<110> STRYKER CORPORATION
<120> METHODS OF TREATING CARTILAGE DEFECTS
<130> STK-13 PCT
<140> Not yet assigned
   <141> 2005-05-24
<160> 11
<170> PatentIn version 3.2
<210> 1
   <211> 431
   <212> PRT
   <213> Homo sapiens
<400> 1
   Met His Val Arg Ser Leu Arg Ala Ala Ala Pro His Ser Phe Val Ala
   1               5                   10                  15
   Leu Trp Ala Pro Leu Phe Leu Leu Arg Ser Ala Leu Ala Asp Phe Ser
               20                  25                  30
   Leu Asp Asn Glu Val His Ser Ser Phe Ile His Arg Arg Leu Arg Ser
           35                  40                  45
   Gln Glu Arg Arg Glu Met Gln Arg Glu Ile Leu Ser Ile Leu Gly Leu
       50                  55                  60
   Pro His Arg Pro Arg Pro His Leu Gln Gly Lys His Asn Ser Ala Pro
   65                  70                  75                  80
   Met Phe Met Leu Asp Leu Tyr Asn Ala Met Ala Val Glu Glu Gly Gly
                   85                  90                  95
   Gly Pro Gly Gly Gln Gly Phe Ser Tyr Pro Tyr Lys Ala Val Phe Ser
               100                 105                 110
   Thr Gln Gly Pro Pro Leu Ala Ser Leu Gln Asp Ser His Phe Leu Thr
           115                 120                 125
   Asp Ala Asp Met Val Met Ser Phe Val Asn Leu Val Glu His Asp Lys
       130                 135                 140
   Glu Phe Phe His Pro Arg Tyr His His Arg Glu Phe Arg Phe Asp Leu
   145                 150                 155                 160
   Ser Lys Ile Pro Glu Gly Glu Ala Val Thr Ala Ala Glu Phe Arg Ile
   165 170 175
   Tyr Lys Asp Tyr Ile Arg Glu Arg Phe Asp Asn Glu Thr Glu Arg Ile
   180 185 190
   Ser Val Tyr Gln Val Leu Gln Glu His Leu Gly Arg Glu Ser Asp Leu
   195 200 205
   Phe Leu Leu Asp Ser Arg Thr Leu Trp Ala Ser Glu Glu Gly Trp Leu
   210 215 220
   Val Phe Asp Ile Thr Ala Thr Ser Asn His Trp Val Val Asn Pro Arg
   225 230 235 240
   His Asn Leu Gly Leu Gln Leu Ser Val Glu Thr Leu Asp Gly Gln Ser
   245 250 255
   Ile Asn Pro Lys Leu Ala Gly Leu Ile Gly Arg His Gly Pro Gln Asn
   260 265 270
   Lys Gln Pro Phe Met Val Ala Phe Phe Lys Ala Thr Glu Val His Phe
   275 280 285
   Arg Ser Ile Arg Ser Thr Gly Ser Lys Gln Arg Ser Gln Asn Arg Ser
   290 295 300
   Lys Thr Pro Lys Asn Gln Glu Ala Leu Arg Met Ala Asn Val Ala Glu
   305 310 315 320
   Asn Ser Ser Ser Asp Gln Arg Gln Ala Cys Lys Lys His Glu Leu Tyr
   325 330 335
   Val Ser Phe Arg Asp Leu Gly Trp Gln Asp Trp Ile Ile Ala Pro Glu
   340 345 350
   Gly Tyr Ala Ala Tyr Tyr Cys Glu Gly Glu Cys Ala Phe Pro Leu Asn
   355 360 365
   Ser Tyr Met Asn Ala Thr Asn His Ala Ile Val Gln Thr Leu Val His
   370 375 380
   Phe Ile Asn Pro Glu Thr Val Pro Lys Pro Cys Cys Ala Pro Thr Gln
   385 390 395 400
   Leu Asn Ala Ile Ser Val Leu Tyr Phe Asp Asp Ser Ser Asn Val Ile
                   405                 410                 415
   Leu Lys Lys Tyr Arg Asn Met Val Val Arg Ala Cys Gly Cys His
               420                 425                 430
<210> 2
   <211> 96
   <212> PRT
   <213> Artificial Sequence
<220>
   <223> Synthetic amino acid sequences COP-5
<400> 2
   Leu Tyr Val Asp Phe Ser Asp Val Gly Trp Asp Asp Trp Ile Val Ala
   1               5                   10                  15
   Pro Pro Gly Tyr Gln Ala Phe Tyr Cys His Gly Glu Cys Pro Phe Pro
               20                  25                  30
   Leu Ala Asp His Phe Asn Ser Thr Asn His Ala Val Val Gln Thr Leu
           35                  40                  45
   Val Asn Ser Val Asn Ser Lys Ile Pro Lys Ala Cys Cys Val Pro Thr
       50                  55                  60
   Glu Leu Ser Ala Ile Ser Met Leu Tyr Leu Asp Glu Asn Glu Lys Val
   65                  70                  75                  80
   Val Leu Lys Tyr Asn Gln Glu Met Val Val Glu Gly Cys Gly Cys Arg
                   85                  90                  95
<210> 3
   <211> 96
   <212> PRT
   <213> Artificial Sequence
<220>
   <223> Synthetic amino acid sequence COP-7
<400> 3
   Leu Tyr Val Asp Phe Ser Asp Val Gly Trp Asn Asp Trp Ile Val Ala
   1               5                   10                  15
   Pro Pro Gly Tyr His Ala Phe Tyr Cys His Gly Glu Cys Pro Phe Pro
               20                  25                  30
   Leu Ala Asp His Leu Asn Ser Thr Asn His Ala Val Val Gln Thr Leu
           35                  40                  45
   Val Asn Ser Val Asn Ser Lys Ile Pro Lys Ala Cys Cys Val Pro Thr
       50                  55                  60
   Glu Leu Ser Ala Ile Ser Met Leu Tyr Leu Asp Glu Asn Glu Lys Val
   65                  70                  75                  80
   Val Leu Lys Tyr Asn Gln Glu Met Val Val Glu Gly Cys Gly Cys Arg
                   85                  90                  95
<210> 4
   <211> 102
   <212> PRT
   <213> Artificial Sequence
<220>
   <223> OP-X
<220>
   <221> MOD_RES
   <222> (1)..(102)
   <223> Xaa is independently selected from a group of one or more specified amino acids as defined in the specification
<220>
   <221> misc_feature
   <222> (2)..(3)
   <223> Xaa can be any naturally occurring amino acid
<220>
   <221> misc_feature
   <222> (9)..(9)
   <223> Xaa can be any naturally occurring amino acid
<220>
   <221> misc_feature
   <222> (11)..(11)
   <223> Xaa can be any naturally occurring amino acid
<220>
   <221> misc_feature
   <222> (16)..(16)
   <223> Xaa can be any naturally occurring amino acid
<220>
   <221> misc_feature
   <222> (19)..(19)
   <223> Xaa can be any naturally occurring amino acid
<220>
   <221> misc_feature
   <222> (23)..(23)
   <223> Xaa can be any naturally occurring amino acid
<220>
   <221> misc_feature
   <222> (26)..(26)
   <223> Xaa can be any naturally occurring amino acid
<220>
   <221> mist_feature
   <222> (35)..(35)
   <223> Xaa can be any naturally occurring amino acid
<220>
   <221> misc_feature
   <222> (39)..(39)
   <223> Xaa can be any naturally occurring amino acid
<220>
   <221> misc_feature
   <222> (41)..(41)
   <223> Xaa can be any naturally occurring amino acid
<220>
   <221> misc_feature
   <222> (50)..(50)
   <223> Xaa can be any naturally occurring amino acid
<220>
   <221> misc_feature
   <222> (52)..(52)
   <223> Xaa can be any naturally occurring amino acid
<220>
   <221> misc_feature
   <222> (56)..(58)
   <223> Xaa can be any naturally occurring amino acid
<220>
   <221> misc_feature
   <222> (60)..(61)
   <223> Xaa can be any naturally occurring amino acid
<220>
   <221> misc_feature
   <222> (65)..(65)
   <223> Xaa can be any naturally occurring amino acid
<220>
   <221> miac_feature
   <222> (71)..(71)
   <223> Xaa can be any naturally occurring amino acid
<220>
   <221> misc_feature
   <222> (73)..(73)
   <223> Xaa can be any naturally occurring amino acid
<220>
   <221> misc_feature
   <222> (75)..(75)
   <223> Xaa can be any naturally occurring amino acid
<220>
   <221> misc_feature
   <222> (80)..(80)
   <223> Xaa can be any naturally occurring amino acid
<220>
   <221> misc_feature
   <222> (82)..(82)
   <223> Xaa can be any naturally occurring amino acid
<220>
   <221> misc_feature
   <222> (84)..(84)
   <223> Xaa can be any naturally occurring amino acid
<220>
   <221> misc_feature
   <222> (87)..(87)
   <223> Xaa can be any naturally occurring amino acid
<220>
   <221> misc_feature
   <222> (89)..(89)
   <223> Xaa can be any naturally occurring amino acid
<220>
   <221> misc_feature
   <222> (91)..(91)
   <223> Xaa can be any naturally occurring amino acid
<220>
   <221> misc_feature
   <222> (97)..(97)
   <223> Xaa can be any naturally occurring amino acid
<400> 4
   Cys Xaa Xaa His Glu Leu Tyr Val Xaa Phe Xaa Asp Leu Gly Trp Xaa
   1               5                   10                  15
   Asp Trp Xaa Ile Ala Pro Xaa Gly Tyr Xaa Ala Tyr Tyr Cys Glu Gly
               20                  25                  30
   Glu Cys Xaa Phe Pro Leu Xaa Ser Xaa Met Asn Ala Thr Asn His Ala
           35                  40                  45
   Ile Xaa Gln Xaa Leu Val His Xaa Xaa Xaa Pro Xaa Xaa Val Pro Lys
       50                  55                  60
   Xaa Cys Cys Ala Pro Thr Xaa Leu Xaa Ala Xaa Ser Val Leu Tyr Xaa
   65                  70                  75                  80
   Asp Xaa Ser Xaa Asn Val Xaa Leu Xaa Lys Xaa Arg Asn Met Val Val
   85 90 95
   Xaa Ala Cys Gly Cys His
   100
<210> 5
   <211> 97
   <212> PRT
   <213> Artificial Sequence
<220>
   <223> Generic Sequence 7
<220>
   <221> MOD_RES
   <222> (1)..(97)
   <223> Xaa is independently selected from a group of one or more specified amino acids as defined in the specification
<220>
   <221> misc_feature
   <222> (2)..(4)
   <223> Xaa can be any naturally occurring amino acid
<220>
   <221> misc_feature
   <222> (6)..(8)
   <223> Xaa can be any naturally occurring amino acid
<220>
   <221> misc_feature
   <222> (11)..(16)
   <223> Xaa can be any naturally occurring amino acid
<220>
   <221> misc_feature
   <222> (18)..(21)
   <223> Xaa can be any naturally occurring amino acid
<220>
   <221> misc_feature
   <222> (23)..(23)
   <223> Xaa can be any naturally occurring amino acid
<220>
   <221> misc_feature
   <222> (26)..(26)
   <223> Xaa can be any naturally occurring amino acid
<220>
   <221> misc_feature
   <222> (28)..(28)
   <223> Xaa can be any naturally occurring amino acid
<220>
   <221> misc_feature
   <222> (30)..(31)
   <223> Xaa can be any naturally occurring amino acid
<220>
   <221> misc_feature
   <222> (33)..(40)
   <223> Xaa can be any naturally occurring amino acid
<220>
   <221> misc_feature
   <222> (44)..(60)
   <223> Xaa can be any naturally occurring amino acid
<220>
   <221> misc_feature
   <222> (63)..(63)
   <223> Xaa can be any naturally occurring amino acid
<220>
   <221> misc_feature
   <222> (65)..(72)
   <223> Xaa can be any naturally occurring amino acid
<220>
   <221> misc_feature
   <222> (74)..(80)
   <223> Xaa can be any naturally occurring amino acid.
<220>
   <221> misc_feature
   <222> (82)..(82)
   <223> Xaa can be any naturally occurring amino acid
<220>
   <221> misc_feature
   <222> (84)..(88)
   <223> Xaa can be any naturally occurring amino acid
<220>
   <221> misc_feature
   <222> (90)..(90)
   <223> Xaa can be any naturally occurring amino acid
<220>
   <221> misc_feature
   <222> (92)..(93)
   <223> Xaa can be any naturally occurring amino acid
<220>
   <221> misc_feature
   <222> (95)..(95)
   <223> Xaa can be any naturally occurring amino acid
<220>
   <221> misc_feature
   <222> (97)..(97)
   <223> Xaa can be any naturally occurring amino acid
<400> 5
   Leu Xaa Xaa Xaa Phe Xaa Xaa Xaa Gly Trp Xaa Xaa Xaa Xaa Xaa Xaa
   1               5                   10                  15
   Pro Xaa Xaa Xaa Xaa Ala Xaa Tyr Cys Xaa Gly Xaa Cys Xaa Xaa Pro
               20                  25                  30
   Xaa Xaa Xaa Xaa Xaa Xaa Xaa Xaa Asn His Ala Xaa Xaa Xaa Xaa Xaa
           35                  40                  45
   Xaa Xaa Xaa Xaa Xaa Xaa Xaa Xaa Xaa Xaa Xaa Xaa Cys Cys Xaa Pro
       50                  55                  60
   Xaa Xaa Xaa Xaa Xaa Xaa Xaa Xaa Leu Xaa Xaa Xaa Xaa Xaa Xaa Xaa
   65                  70                  75                  80
   Val Xaa Leu Xaa Xaa Xaa Xaa Xaa Met Xaa Val Xaa Xaa Cys Xaa Cys
                   85                  90                  95
   Xaa
<210> 6
   <211> 102
   <212> PRT
   <213> Artificial Sequence
<220>
   <223> Generic Sequence 8
<220>
   <221> MOD_RES
   <222> (1)..(102)
   <223> Xaa is independently selected from a group of one or more specified amino acids as defined in the specification
<220>
   <221> misc_feature
   <222> (2)..(5)
   <223> Xaa can be any naturally occurring amino acid
<220>
   <221> misc_feature
   <222> (7)..(9)
   <223> Xaa can be any naturally occurring amino acid
<220>
   <221> misc_feature
   <222> (11)..(13)
   <223> Xaa can be any naturally occurring amino acid
<220>
   <221> misc_feature
   <222> (16)..(21)
   <223> Xaa can be any naturally occurring amino acid
<220>
   <221> misc_feature
   <222> (23)..(26)
   <223> Xaa can be any naturally occurring amino acid
<220>
   <221> misc_feature
   <222> (28)..(28)
   <223> Xaa can be any naturally occurring amino acid
<220>
   <221> misc_feature
   <222> (31)..(31)
   <223> Xaa can be any naturally occurring amino acid
<220>
   <221> misc_feature
   <222> (33)..(33)
   <223> Xaa can be any naturally occurring amino acid
<220>
   <221> misc_feature
   <222> (35)..(36)
   <223> Xaa can be any naturally occurring amino acid
<220>
   <221> misc_feature
   <222> (38)..(45)
   <223> Xaa can be any naturally occurring amino acid
<220>
   <221> misc_feature
   <222> (49)..(65)
   <223> Xaa can be any naturally occurring amino acid
<220>
   <221> misc_feature
   <222> (68)..(68)
   <223> Xaa can be any naturally occurring amino acid
<220>
   <221> misc_feature
   <222> (70)..(77)
   <223> Xaa can be any naturally occurring amino acid
<220>
   <221> misc_feature
   <222> (79)..(85)
   <223> Xaa can be any naturally occurring amino acid
<220>
   <221> misc_feature
   <222> (87)..(87)
   <223> Xaa can be any naturally occurring amino acid
<220>
   <221> misc_feature
   <222> (89).. (93)
   <223> Xaa can be any naturally occurring amino acid
<220>
   <221> misc_feature
   <222> (95)..(95)
   <223> Xaa can be any naturally occurring amino acid
<220>
   <221> misc_feature
   <222> (97)..(98)
   <223> Xaa can be any naturally occurring amino acid
<220>
   <221> misc_feature
   <222> (100)..(100)
   <223> Xaa can be any naturally occurring amino acid
<220>
   <221> misc feature
   <222> (102)..(102)
   <223> Xaa can be any naturally occurring amino acid
<400> 6
   Cys Xaa Xaa Xaa Xaa Leu Xaa Xaa Xaa Phe Xaa Xaa Xaa Gly Trp Xaa
   1               5                   10                  15
   Xaa Xaa Xaa Xaa Xaa Pro Xaa Xaa Xaa Xaa Ala Xaa Tyr Cys Xaa Gly
               20                  25                  30
   Xaa Cys Xaa Xaa Pro Xaa Xaa Xaa Xaa Xaa Xaa Xaa Xaa Asn His Ala
           35                  40                  45
   Xaa Xaa Xaa Xaa Xaa Xaa Xaa Xaa Xaa Xaa Xaa Xaa Xaa Xaa Xaa Xaa
       50                  55                  60
   Xaa Cys Cys Xaa Pro Xaa Xaa Xaa Xaa Xaa Xaa Xaa Xaa Leu Xaa Xaa
   65                  70                  75                  80
   Xaa Xaa Xaa Xaa Xaa Val Xaa Leu Xaa Xaa Xaa Xaa Xaa Met Xaa Val
                   85                  90                  95
   Xaa Xaa Cys Xaa Cys Xaa
               100
<210> 7
   <211> 97
   <212> PRT
   <213> Artificial Sequence
<220>
   <223> Generic Sequence 9
<220>
   <221> MOD_RES
   <222> (1)..(97)
   <223> Xaa is independently selected from a group of one or more specified amino acids as defined in the specification
<220>
   <221> misc_feature
   <222> (1)..(16)
   <223> Xaa can be any naturally occurring amino acid
<220>
   <221> misc_feature
   <222> (18)..(24)
   <223> Xaa can be any naturally occurring amino acid
<220>
   <221> misc_feature
   <222> (26)..(26)
   <223> Xaa can be any naturally occurring amino acid
<220>
   <221> misc_feature
   <222> (28)..(28)
   <223> Xaa can be any naturally occurring amino acid
<220>
   <221> misc_feature
   <222> (30)..(61)
   <223> Xaa can be any naturally occurring amino acid
<220>
   <221> misc_feature
   <222> (63)..(63)
   <223> Xaa can be any naturally occurring amino acid
<220>
   <221> misc_feature
   <222> (65)..(72)
   <223> Xaa can be any naturally occurring amino acid
<220>
   <221> misc_feature
   <222> (74)..(93)
   <223> Xaa can be any naturally occurring amino acid
<220>
   <221> misc_feature
   <222> (95)..(95)
   <223> Xaa can be any naturally occurring amino acid
<220>
   <221> misc_feature
   <222> (97)..(97)
   <223> Xaa can be any naturally occurring amino acid
<400> 7
   Xaa Xaa Xaa Xaa Xaa Xaa Xaa Xaa Xaa Xaa Xaa Xaa Xaa Xaa Xaa Xaa
   1               5                   10                  15
   Pro Xaa Xaa Xaa Xaa Xaa Xaa Xaa Cys Xaa Gly Xaa Cys Xaa Xaa Xaa
               20                  25                  30
   Xaa Xaa Xaa Xaa Xaa Xaa Xaa Xaa Xaa Xaa Xaa Xaa Xaa Xaa Xaa Xaa
           35                  40                  45
   Xaa Xaa Xaa Xaa Xaa Xaa Xaa Xaa Xaa Xaa Xaa Xaa Xaa Cys Xaa Pro
       50                  55                  60
   Xaa Xaa Xaa Xaa Xaa Xaa Xaa Xaa Leu Xaa Xaa Xaa Xaa Xaa Xaa Xaa
   65                  70                  75                  80
   Xaa Xaa Xaa Xaa Xaa Xaa Xaa Xaa Xaa Xaa Xaa Xaa Xaa Cys Xaa Cys
                   85                  90                  95
   Xaa
<210> 8
   <211> 102
   <212> PRT
   <213> Artificial Sequence
<220>
   <223> Generic Sequence 10
<220>
   <221> MOD_RES
   <222> (1)..(102)
   <223> Xaa is independently selected from a group of one or more specified amino acids as defined in the specification
<220>
   <221> misc_feature
   <222> (2)..(21)
   <223> Xaa can be any naturally occurring amino acid
<220>
   <221> misc_feature
   <222> (23)..(29)
   <223> Xaa can be any naturally occurring amino acid
<220>
   <221> misc_feature
   <222> (31)..(31)
   <223> Xaa can be any naturally occurring amino acid
<220>
   <221> misc_feature
   <222> (33)..(33)
   <223> Xaa can be any naturally occurring amino acid
<220>
   <221> misc_feature
   <222> (35)..(66)
   <223> Xaa can be any naturally occurring amino acid
<220>
   <221> misc_feature
   <222> (68)..(68)
   <223> Xaa can be any naturally occurring amino acid
<220>
   <221> misc_feature
   <222> (70)..(77)
   <223> Xaa can be any naturally occurring amino acid
<220>
   <221> misc_feature
   <222> (79)..(98)
   <223> Xaa can be any naturally occurring amino acid
<220>
   <221> misc_feature
   <222> (100)..(100)
   <223> Xaa can be any naturally occurring amino acid
<220>
   <221> misc_feature
   <222> (102)..(102)
   <223> Xaa can be any naturally occurring amino acid
<400> 8
   Cys Xaa Xaa Xaa Xaa Xaa Xaa Xaa Xaa Xaa Xaa Xaa Xaa Xaa Xaa Xaa
   1               5                   10                  15
   Xaa Xaa Xaa Xaa Xaa Pro Xaa Xaa Xaa Xaa Xaa Xaa Xaa Cys Xaa Gly
               20                  25                  30
   Xaa Cys Xaa Xaa Xaa Xaa Xaa Xaa Xaa Xaa Xaa Xaa Xaa Xaa Xaa Xaa
           35                  40                  45
   Xaa Xaa Xaa Xaa Xaa Xaa Xaa Xaa Xaa Xaa Xaa Xaa Xaa Xaa Xaa Xaa
       50                  55                  60
   Xaa Xaa Cys Xaa Pro Xaa Xaa Xaa Xaa Xaa Xaa Xaa Xaa Leu Xaa Xaa
   65                  70                  75                  80
   Xaa Xaa Xaa Xaa Xaa Xaa Xaa Xaa Xaa Xaa Xaa Xaa Xaa Xaa Xaa Xaa
                   85                  90                  95
   Xaa Xaa Cys Xaa Cys Xaa
   100
<210> 9
   <211> 5
   <212> PRT
   <213> Artificial Sequence
<220>
   <223> Consensus Sequence
<220>
   <221> MOD_RES
   <222> (1)..(5)
   <223> Xaa is independently selected from a group of one or more specified amino acids as defined in the specification
<220>
   <221> misc_feature
   <222> (2)..(5)
   <223> Xaa can be any naturally occurring amino acid
<400> 9
   Cys Xaa Xaa Xaa Xaa
   1               5
<210> 10
   <211> 1822
   <212> DNA
   <213> Homo sapiens
<220>
   <221> CDS
   <222> (49)..(1341)
<400> 10
<2.0> 11 <211> 431
   <212> PRT
   <213> Homo sapiens
<400> 11

## Description

### FIELD OF THE INVENTION

The present invention relates to orthopaedic tissue repair. More particularly, it relates to repairing or regenerating cartilage.

### BACKGROUND OF THE TNVENTION

Cartilage repair and regeneration is one of the major obstacles in current orthopedics. The importance is enormous because cartilage injury and degenerative disorders such as osteoarthritis, intervertebral disc degeneration and meniscal tears are a major cause of disability among the adult population in the United States.

Cartilage is connective tissue composed of chondrocytes embedded in an extracellular matrix of collagen fibers, proteoglycans, and other non-collagenous proteins. There are two forms of cartilage- articular and non-articular. Articular cartilage is a thin layer of connective tissue, which covers the ends of bones in joints. Non-articular cartilage includes fibrocartilage and elastic cartilage and includes intervertebral discs, meniscus, trachea, larynx, nose, ear and ribs.

The function of cartilage is to cushion load bearing, resist wear, and allow for almost frictionless movement of joints. Defects in cartilage tissue, often caused by trauma, abnormal wear or disease, can lead to pain and stiffness, and if left untreated, may progress and ultimately require replacement of the entire joint. For example, articular cartilage defects often lead to early degradation of the articular surface and may eventually result in osteochondral defects, osteoarthritis or both.

Osteoarthritis is considered a process of attempted, but gradually failing, repair of damaged cartilage extracellular matrix, as the balance between synthesis and breakdown of matrix components is disturbed and shifted toward catabolism.

The ability of cartilage tissue to regenerate on its own is severely limited due to its avascular nature. Repair of osteochondral defects, which involves both the cartilage tissue and the underlying bone, occurs to a limited extent promoted by the presence of both stem cells and growth and differentiation factors brought into the defect by the blood and/or marrow. In animal studies, these defects undergo some repair with formation of a new layer of bone and cartilage, but the macromolecular organization and the biochemical characteristics of the cartilage matrix are imperfect. Type I collagen, rather than Type II collagen, and proteoglycans that are not cartilage specific, such as dermatan sulfate containing proteoglycans, make up the repair tissue and result in fibrillations and degenerative changes over time. And, repair of cartilage defects that do not penetrate into the subchondral bone does not occur, even to a limited extent.

Moreover, surgical treatment of cartilage defects is complex and has been demonstrated to have only limited success. For example, articular cartilage defects are treated with an arthroscopic approach where loose bodies are debrided and transition areas are smoothed. However, this method alone frequently does not provide long lasting relief of the symptoms. Knee replacements often require resecting significant amounts of bone and often require multiple surgeries.

The meniscus is a small horseshoe shaped tissue located between the bone ends inside the knee joint, which acts as a shock absorber. There are two menisci in each knee on either side of the knee. They are usually strong in young people and with age become more brittle and tear more easily. Tears are extremely common with anterior cruciate ligament (ACL) injuries. Meniscal fibrocartilage, like articular hyaline cartilage, has a limited capacity to heal, particularly in the middle and inner avascular regions. The current treatment for small tears is to leave them alone if they do not cause much trouble. Surgical options for treating meniscal tears depend on a number of factors including the nature and extent of the injury and most importantly, its location. Tears in the vascularized region, which is integrated with the highly vascularized synovium have been successfully repaired by suturing. Partial or total meniscectomy is the normal surgical treatment for symptomatic tears within the avascular two thirds of the meniscus. Tears in the latter meniscus regions are the most common types seen clinically. Irrespective of whether open, arthroscopic, total or partial meniscectomy are employed, osteoarthritis is a frequent sequela in these patients within a few years post surgery. Therefore, the common form of repair is to only partially remove the torn bits and to repair the cartilage by stapling it. Unfortunately, the healing process following this procedure is slow. Moreover, if the repair is not successful, then the entire torn meniscus must subsequently be removed.

The major cause of persistent and often debilitating back pain is intervertebral disc (IVD) degeneration. As discs degenerate, they cause the adjoining vertebrae to become compressed, often resulting in severe pain.

The IVD as a syndesmosis provides articulation between adjoining vertebral bodies and acts as a weight bearing cushion which dissipates axially applied spinal loads. These biomechanical functions are made possible by the unique structure of the IVD which is composed of an outer collagen-rich annulus fibrosus surrounding a central hydrated proteoglycan rich gelatinous nucleus pulposus. Superior and inferior cartilaginous endplates, thin layers of hyaline-like cartilage covers the interfaces of the vertebral bodies within the disc.

Lumbar disc degeneration represents a substantial social and economic burden to the community which is manifest principally as low back pain (LBP). It is estimated that as much as 80% of the population experience at least one significant episode of LBP during life, and approximately 2.5% of the working population will take some sick leave during the year as a result of LBP. The direct costs of LBP in modem Western countries has been estimated at $9 billion, most of which is spent on consulting general practitioners, physical therapists and other conservative practitioners (Williams DA et al., (1998) Health care and indemnity costs across the natural history of disability in occupational low back pain, Spine 23:2329-36). Total indirect expenditure, including surgical management may be ten times higher (Maetzel and Li (2002) The economic burden of low back pain: a review of studies published between 1996 and 2001, Best Prac Res Clin Rheumatol 16:23-30; Walker et al., (2003) The economic burden, Proceedings of the Spine Society of Australia Annual Scientific Meeting, Canberra, Australia).

Disc degeneration is a natural phenomenon that occurs, in most instances, from the time of skeletal maturity (Vernon-Roberts (1992) Age-related and degenerative pathology of intervertebral discs and apophyseal joints, In: The lumbar spine and back pain. Fourth edition, Jayson MIX, Ed. Churchill Livingstone, Edinburgh, Chapter 2, 17-41). It is consistent with advancing age but in many cases is also associated with pain, particularly in the lumbar spine, and restricted mobility. Symptoms of LBP often resolve spontaneously over time as patients modify their lifestyles to accommodate restricted mobility. In many cases however, it remains a significant factor that requires surgical intervention. The traditional "gold standard" surgical treatment for chronic LBP has been spinal fusion to immobilize the one or more painful level. Fusion is expensive because it requires prolonged hospitalization and specialist surgical expertise, and although most of these patients will experience short-term pain relief there is evidence now that fusion does not provide the best outcome. Long-term studies suggest that spinal fusion actually promotes degeneration at levels adjacent to the fusion site (Lee (1988) Accelerated degeneration of the segment adjacent to a lumbar fusion, Spine 13:375-7.). In the same way that artificial prostheses were developed 50 years ago to restore function to arthritic and fractured hips and knees, prostheses are now being developed with the aim of restoring full mechanical function to discs that have become painful and arthritic due to chronic degeneration (Szpaalski et al (2002) V Spine arthroplasty: a historical review, Eur Spine J 11:565-S84). It is however too early to know if any of the myriad models undergoing trials will provide long-term benefit.

A class of proteins have now been identified that are competent to act as true bone and cartilage tissue morphogens, able, on their own, to induce the proliferation and differentiation of progenitor cells into functional bone, cartilage, tendon, and/or ligamentous tissue. These proteins, referred to herein as "osteogenic proteins" or "morphogenic proteins" or "morphogens," includes members of the family of bone morphogenetic proteins (BMPs) which were initially identified by their ability to induce ectopic, endochondral bone morphogenesis. The osteogenic proteins generally are classified in the art as a subgroup of the TGF-β superfamily of growth factors (Hogan (1996) Genes & Development 10:1580-1594). Members of the morphogen family of proteins include the mammalian osteogenic protein-1 (OP-1, also known as BMP-7, and the *Drosophila* homolog 60A), osteogenic protein-2 (OP-2, also known as BMP-8), osteogenic protein-3 (OP-3), BMP-2 (also known as BMP-2A or CBMP-2A, and the *Drosophila* homolog DPP), BMP-3, BMP-4 (also known as BMP-2B or CBMP-2B), BMP-5, BMP-6 and its murine homolog Vgr-1, BMP-9, BMP-10, BMP-11, BMP-12, GDF3 (also known as Vgr2), GDF8, GDF9, GDF10, GDF11, GDF12, BMP-13, BMP-14, BMP-15, BMP-16, Bump-17, BMP-18, GDF-5 (also known as CDMP-1 or MP52), GDF-6 (also known as CDMP-2), GDF-7 (also known as CDMP-3), the *Xenopus* homolog Vg1 and NODAL, UNIVIN, SCREW, ADMP, and NEURAL. Members of this family encode secreted polypeptide chains sharing common structural features, including processing from a precursor "pro-form" to yield a mature polypeptide chain competent to dimerize, and containing a carboxy terminal active domain of approximately 97-106 amino acids. All members share a conserved pattern of cysteines in this domain and the active form of these proteins can be either a disulfide-bonded homodimer of a single family member, or a heterodimer of two different members (see, e.g., Massague (1990) Annu. Rev. Cell Biol. 6:597; Sampath, et al. (1990) J. Biol. Chem. 265:13198). See also, U.S. 5,011,691; U.S. 5,266,683, Ozkaynak et al. (1990) EMBO J. 9: 2085-2093, Wharton et al. (1991) PNAS 88:9214-9218), (Ozkaynak (1992) J. Biol. Chem. 267:25220-25227 and U.S. 5,266,683); (Celeste et al. (1991) PNAS 87:9843-9847); (Lyons et al. (1989) PNAS 86:4554-4558). These disclosures describe the amino acid and DNA sequences, as well as the chemical and physical characteristics of these osteogenic proteins. See also Wozney et al. (1988) Science 242:1528-1534); BMP 9 (WO93/00432, published January 7, 1993); DPP (Padgett et al. (1987) Nature 325:81-84; and Vg-1 (Weeks (1987) Cell 51:861-867).

The currently preferred methods of repairing cartilage defects include debridement, microfracture, autologous cell transplantation, mosaicplasty and joint replacement. However, none of these methods, result in actual repair and replacement of cartilage tissue. These methods result in imperfect repair tissue with scar-like characteristics.

Therefore, there remains a need for compositions and methods for repairing and regenerating cartilage defects which overcome the problems associated with the currently available methods and compositions.

### SUMMARY OF THE INVENTION

The present invention relates to a pharmaceutical composition consisting of OP-1 and a pharmaceutically acceptable carrier for use in repairing an articular cartilage defect in a patient wherein the cartilage is surrounded by synovial fluid; wherein the pharmaceutically acceptable carrier is selected from water, saltine, Ringer's solution, a buffered solution, hyaluronan and dextrose solution; wherein the pharmaceutical composition is a liquid solution or liquid suspension; and wherein the pharmaceutical composition is to be administered into the synovial fluid surrounding the articular cartilage.
The present invention also relates to a pharmaceutical composition consisting of OP-1 and a pharmaceutically acceptable carrier for use in regenerating or producing articular cartilage at a cartilage defect site in a patient wherein the articular cartilage is surrounded by synovial fluid; wherein the pharmaceutically acceptable carrier is selected from water, saline, Ringer's solution, a buffered solution, hyaluronan and dextrose solution; wherein the pharmaceutical composition is a liquid solution or liquid suspension; and wherein the pharmaceutical composition is to be administered into the synovial fluid surrounding the articular cartilage.
The present invention furthermore relates to a pharmaceutical composition consisting of OP-1 and a pharmaceutically acceptable carrier for use in promoting articular cartilage growth or accelerating articular cartilage : formation at a cartilage defect site in a patient wherein the articular cartilage is surrounded by synovial fluid; wherein the pharmaceutically acceptable carrier is selected from water, saline, Ringer's solution, a buffered solution, hyaluronan and dextrose solution; wherein the pharmaceutical composition is a liquid solution or liquid suspension; and wherein the pharmaceutical composition is to be administered into the synovial fluid surrounding the articular cartilage.
The present invention also relates to a pharmaceutical composition consisting of OP-1 and a pharmaceutically acceptable carrier for use in preventing articular cartilage degradation or treating articular cartilage injury or degenerative disease or disorder in a patient wherein the articular cartilage is surrounded by synovial fluid; wherein the pharmaceutically acceptable carrier is selected from water, saline, Ringer's solution, a buffered solution, hyaluronan and dextrose solution; wherein the pharmaceutical composition is a liquid solution or liquid suspension; and wherein the pharmaceutical composition is to be administered into the synovial fluid surrounding the articular cartilage.

In some embodiments the tissue injury or degenerative disease includes osteoarthritis, meniscus tears, ACL injury and disc degeneration.

In some embodiments, the cartilage is articular cartilage. In other embodiments, the cartilage is non-articular cartilage. In some embodiments, the non-articular cartilage is a meniscus or an intervertebral disc.

In some embodiments, the OP-1 composition used in the methods of this invention includes a gel, a putty, a paste or an aqueous solution. In some embodiments, the OP-1 composition is formulated as a sustained release formulation or as a delayed clearance formulation (i.e., a formulation whereby the clearance of the morphogenic protein is delayed relative to its normal clearance). In some embodiments, the OP-1 composition is formulated as an injectable formulation

### BRIEF DESCRIPTION OF THE DRAWINGS

Figure 1 is a schematic of a joint showing the site of the bilateral impact. injuries.
Figure 2 is a graph showing the number of leucocytes in the synovial fluid for OP-1-treated and control animals.
Figures 3A and 3B are histological sections of control and OP-1-treated joints.
Figure 4 is a graph showing the sGAG levels in medial femoral condyle cartilage for OP-1-treated and control animals.
Figure 5 shows representative results of control and OP-I-treated sheep in the osteoarthritis model. Control sheep were treated with collagen alone. OP-1-treated sheep received 350 µg OP-1 putty at the time of surgery and a second dose was injected into the joint space 1 week later.
Figure 6 is a histological section of hole 6 weeks after treatment with OP-1 putty.
Figure 7 is a histological section of hole 6 week control defect.
Figure 8 is a histological section of hole 12 week control defect.
Figure 9 is a histological section of hole 12 weeks after treatment with OP-1 putty.
Figure 10 is a histological section of a meniscal tear defect 6 weeks after treatment with OP-1 putty.

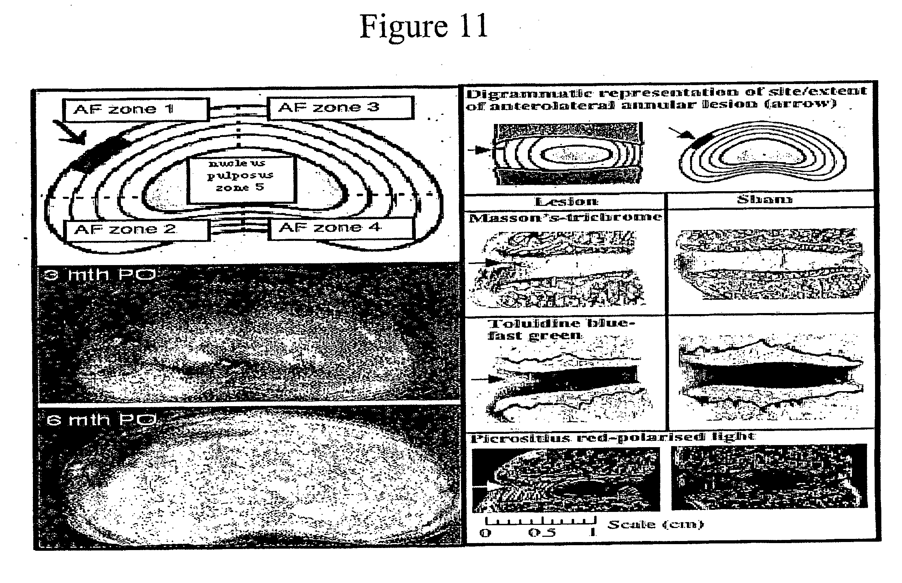

Figure 11 depicts the zonal dissection scheme to separate the disc into annulus fibrosus (AF) quadrants and the nucleus pulposus (NP) and the location and extent of the anterolateral annular lesion in quadrant 1 in horizontal and vertical sections through lumbar ovine intervertebral discs. The location of the AF lesion in horizontal sections 3 and 6 months post surgery is well illustrated on the left hand side of this figure. Vertical histological sections through the intervertebral disc and adjacent vertebral body and superior and inferior vertebral growth plates also demonstrates the focal nature of the AF lesion (arrow) associated with changes in collagen in the outer AF (Masson trichrome) and collagenous organization (picrosirius red) and focal depletion of proteoglycan (toluidine blue) in the lesion site which penetrates approximately 4mm into the disc (right hand side of figure).
Figure 12 depicts flexion-extension ROM plots for intact and injured (anterior annular lesion) sheep functional spinal unit (FSU).

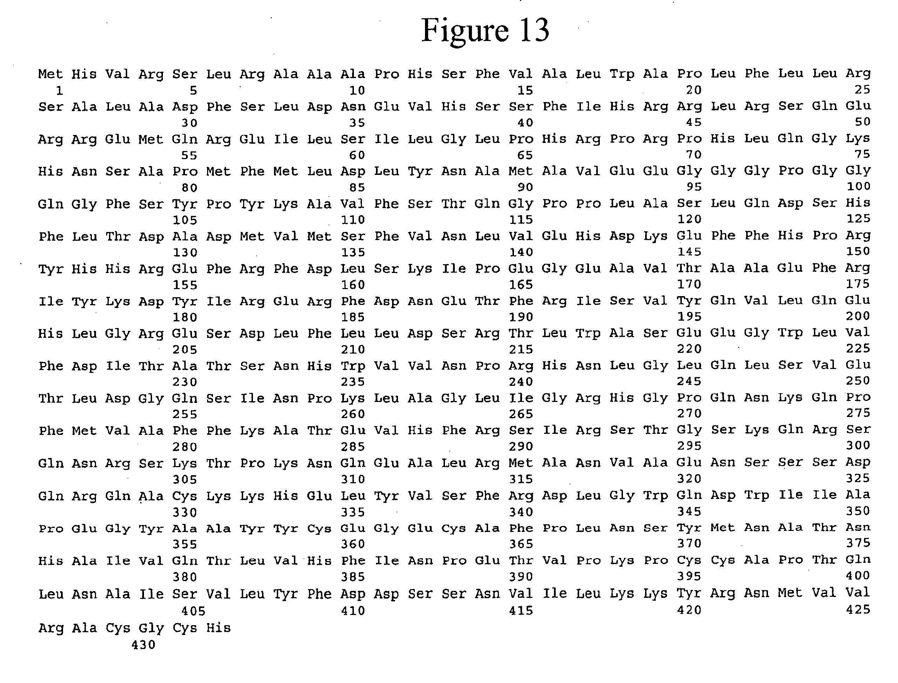

Figure 13 depicts the amino acid sequence of human OP-1.

### DETAILED DESCRIPTION OF THE INVENTION

In order that the invention herein described may be fully understood, the following detailed description is set forth.

Unless defined otherwise, all technical and scientific terms used herein have the same meaning as those commonly understood by one of ordinary skill in the art to which this invention belongs. Although methods and materials similar or equivalent to those described herein can be used in the practice or testing of the present invention, suitable methods and materials are described below. The materials, methods and examples are illustrative only, and are not intended to be limiting.

Throughout this specification, the word "comprise" or variations such as "comprises" or "comprising" will be understood to imply the inclusion of a stated integer or groups of integers but not the exclusion of any other integer or group of integers.

In order to further define the invention, the following terms and definitions are provided herein.

The term "cartilage" refers to a type of connective tissue that contains chondrocytes or chondrocyte-like cells (having many, but not all characteristics of chondrocytes) and intercellular material (e.g., Types I, II, IX and XI collagen), proteoglycans (e.g., chondroitin sulfate, keratan sulfate, and dermatan sulfate proteoglycans) and other proteins. Cartilage includes articular and non-articular cartilage.

"Articular cartilage," also referred to as hyaline cartilage, refers to an avascular, non-mineralized connective tissue, which covers the articulating surfaces of bones in joints and serves as a friction reducing interface between two opposing bone surfaces. Articular cartilage allows movement in joints without direct bone-to-bone contact. Articular cartilage has no tendency to ossification. The cartilage surface appears smooth and pearly macroscopically, and is finely granular under high power magnification. Articular cartilage derives nutrients partly from the vessels of the neighboring synovial membrane and partly from the vessels of the bone it covers. Articular cartilage is associated with the presence of Type II and Type IX collagen and various well-characterized proteoglycans, and with the absence of Type X collagen, which is associated with endochondral bone formation. For a detailed description of articular cartilage microstructure, see, for example, Aydelotte and Kuettner, Conn. Tiss. Res., 18, p. 205 (1988); Zanetti et al., J. Cell Biol., 101, p. 53 (1985); and Poole et al., J. Anat., 138, p. 13 (1984).

"Non-articular cartilage" refers to cartilage that does not cover articulating surfaces and includes fibrocartilage (including interarticular fibrocartilage, fibrocartilaginous disc, connecting fibrocartilage and circumferential fibrocartilage) and elastic cartilage. In fibrocartilage, the micropolysaccharide network is interlaced with prominent collagen bundles, and the chondrocytes are more widely scattered than in hyaline or articular cartilage. Interarticular fibrocartilage is found in joints which are exposed to concussion and subject to frequent movement, e.g., the meniscus of the knee. Examples of such joints include but are not limited to the temporo-mandibular, sterno-clavicular, acromio-clavicular, wrist and knee joints. Secondary cartilaginous joints are formed by discs of fibrocartilage. Such fibrocartilaginous discs, which adhere closely to both of the opposed surfaces, are composed of concentric rings of fibrous tissue, with cartilaginous laminae interposed. An example of such fibrocartilaginous disc is the intervertebral disc of the spine. Connecting fibrocartilage is interposed between the bony surfaces of those joints, which allow for slight mobility as between the bodies of the vertebrae and between the pubic bones. Circumferential fibrocartilage surrounds the margin of some of the articular cavities, such as the cotyloid cavity of the hip and the glenoid cavity of the shoulder.

Elastic cartilage contains fibers of collagen that are histologically similar to elastin fibers. Such cartilage is found in the auricle of the external ear, the eustachian tubes, the cornicula laryngis and the epiglottis. As with all cartilage, elastic cartilage also contains chondrocytes and a matrix, the latter being pervaded in every direction, by a network of yellow elastic fibers, branching and anastomosing in all directions except immediately around each cell, where there is a variable amount of non-fibrillated, hyaline, intercellular substance.

The term "synovial fluid" refers to a thin, lubricating substance within the synovial cavity that reduces friction within the joint.

The term "defect" or "defect site", refers to a disruption of chondral or osteochondral tissue. A defect can assume the configuration of a "void", which is understood to mean a three-dimensional defect such as, for example, a gap, cavity, hole or other substantial disruption in the structural integrity of chondral or osteochondral tissue. A defect can also be a detachment of the cartilage from its point of attachment to the bone or ligaments. In certain embodiments, the defect is such that it is incapable of endogenous or spontaneous repair. A defect can be the result of accident, disease, and/or surgical manipulation. For example, cartilage defects may be the result of trauma to a joint such as a displacement of torn meniscus tissue into the joint. Cartilage defects may be also be the result of degenerative joint diseases such as osteoarthritis.

The term "repair" refers to new cartilage formation which is sufficient to at least partially fill the void or structural discontinuity at the defect site. Repair does not, however, mean, or otherwise necessitate, a process of complete healing or a treatment, which is 100% effective at restoring a defect to its pre-defect physiological/structural/mechanical state.

The term "therapeutically effective amount" refers to an amount effective to repair, regenerate, promote, accelerate, prevent degradation, or form cartilage tissue.

The term "patient" refers to an animal including a mammal (e.g., a human).

The term "morphogenic protein" refers to a protein having morphogenic activity. Preferably a morphogenic protein of this invention comprises at least one polypeptide belonging to the BMP protein family. Morphogenic proteins include osteogenic proteins. Morphogenic proteins may be capable of inducing progenitor cells to proliferate and/or to initiate differentiation pathways that lead to cartilage, bone, tendon, ligament or other types of tissue formation depending on local environmental cues, and thus morphogenic proteins may behave differently in different surroundings. For example, a morphogenic protein may induce bone tissue at one treatment site and cartilage tissue at a different treatment site.

The term "bone morphogenic protein (BMP)" refers to a protein belonging to the BMP family of the TGF-β superfamily of proteins (BMP family) based on DNA and amino acid sequence homology. A protein belongs to the BMP family according to this invention when it has at least 50% amino acid sequence identity with at least one known BMP family member within the conserved C-terminal cysteine-rich domain, which characterizes the BMP protein family. Preferably, the protein has at least 70% amino acid sequence identity with at least one known BMP family member within the conserved C-terminal cysteine rich domain. Members of the BMP family may have less than 50% DNA or amino acid sequence identity overall. Osteogenic protein as defined herein also is competent to induce articular cartilage formation at an appropriate in vivo avascular locus.

The term "amino acid sequence homology" is understood to include both amino acid sequence identity and similarity. Homologous sequences share identical and/or similar amino acid residues, where similar residues are conservative substitutions for, or "allowed point mutations" of, corresponding amino acid residues in an aligned reference sequence. Thus, a candidate polypeptide sequence that shares 70% amino acid homology with a reference sequence is one in which any 70% of the aligned residues are either identical to, or are conservative substitutions of, the corresponding residues in a reference sequence. Certain particularly preferred morphogenic polypeptides share at least 60%, and preferably 70% amino acid sequence identity with the C-terminal 102-106 amino acids, defining the conserved seven-cysteine domain of human OP-1 and related proteins.

Amino acid sequence homology can be determined by methods well known in the art. For instance, to determine the percent homology of a candidate amino acid sequence to the sequence of the seven-cysteine domain, the two sequences are first aligned. The alignment can be made with, e.g., the dynamic progamming algorithm described in Needleman et al., J. Mol. Biol., 48, pp. 443 (1970), and the Align Program, a commercial software package produced by DNAstar, Inc.
An initial alignment can be refined by comparison to a multi-sequence alignment of a family of related proteins. Once the alignment is made and refined, a percent homology score is calculated. The aligned amino acid residues of the two sequences are compared sequentially for their similarity to each other. Similarity factors include similar size, shape and electrical charge. One particularly preferred method of determining amino acid similarities is the PAM250 matrix described in Dayhoff et al., Atlas of Protein Sequence and Structure, 5, pp. 345-352 (1978 & Supp.). A similarity score is first calculated as the sum of the aligned pair wise amino acid similarity scores. Insertions and deletions are ignored for the purposes of percent homology and identity. Accordingly, gap penalties are not used in this calculation. The raw score is then normalized by dividing it by the geometric mean of the scores of the candidate sequence and the seven-cysteine domain. The geometric mean is the square root of the product of these scores. The normalized raw score is the percent homology.

The term "conservative substitutions" refers to residues that are physically or functionally similar to the corresponding reference residues. That is, a conservative substitution and its reference residue have similar size, shape, electric charge, chemical properties including the ability to form covalent or hydrogen bonds, or the like. Preferred conservative substitutions are those fulfilling the criteria defined for an accepted point mutation in Dayhoff *et al., supra.* Examples of conservative substitutions are substitutions within the following groups: (a) valine, glycine; (b) glycine, alanine; (c) valine, isoleucine, leucine; (d) aspartic acid, glutamic acid; (e) asparagine, glutamine; (f) serine, threonine; (g) lysine, arginine, methionine; and (h) phenylalanine, tyrosine. The term "conservative variant" or "conservative variation" also includes the use of a substituting amino acid residue in place of an amino acid residue in a given parent amino acid sequence, where antibodies specific for the parent sequence are also specific for, i.e., "cross-react" or "immuno-react" with, the resulting substituted polypeptide sequence.

The term "osteogenic protein (OP)" refers to a morphogenic protein that is capable of inducing a progenitor cell to form cartilage and/or bone. The bone may be intramembranous bone or endochondral bone. Most osteogenic proteins are members of the BMP protein family and are thus also BMPs. As described elsewhere herein, the class of proteins is typified by human osteogenic protein (hOP-1). The osteogenic protein useful in the practice of the invention is an osteogenically active form of OP-1, and amino acid sequence variants thereof. OP-1 suitable for use with applicants' invention can be identified by means of routine experimentation using the art-recognized bioassay described by Reddi and Sampath (Sampath et al., Proc: Natl. Acad. Sci., 84, pp. 7109-13).

### Compositions For Cartilage and Repair and Their Uses

The compositions may be used for cartilage repair (e.g., at a joint, meniscus or intervertebral disc). The compositions comprising. OP-1 disclosed herein will permit the physician to treat a variety of tissue injuries, tissue degenerative or disease conditions and disorders that can be ameliorated or remedied by localized, stimulated tissue regeneration or repair.

The invention provides, compositions for use in treating cartilage tissue injuries and cartilage degenerative diseases or disorders including but not limited to osteoarthritis, meniscus tears, ACL injuries and disc degeneration.

In some embodiments, the invention provides compositions for use in repairing or regenerating cartilage at a cartilage defect site in a patient. The invention also provides compositions for use in producing cartilage, promoting cartilage growth accelerating cartilage formation at a cartilage defect site and for use in preventing cartilage degradation in a patient.

The composition for use of the present invention is destined to be administered into the synovial fluid surrounding the cartilage. In some embodiments, the cartilage is articular cartilage. In other embodiments, the cartilage is non-articular cartilage. In some embodiments, the non-articular cartilage includes but is not limited to intervertebral disc, interarticular meniscus.

### Bone Morphogenic Protein Family

The BMP family, named for its representative bone morphogenic/osteogenic protein family members, belongs to the TGF-β protein superfamily. Of the reported "BMPs" (BMP-1 to BMP-18), isolated primarily based on sequence homology, all but BMP-L remain classified as members of the BMP family of morphogenic proteins (Ozkaynak et al., EMBO J., 9, pp. 2085-93 (1990)).

The BMP family includes other structurally-related members which are morphogenic proteins, including the *drosophila* decapentaplegic gene complex (DPP) products, the Vgl product of *Xenopus laevis* and its murine homolog, Vgr-1 (see, *e.g*., Massagué, Annu. Rev. Cell Biol., 6, pp. 597-641 (1990).

The C-terminal domains of BMP-3, BMP-5, BMP-6, and OP-1 (BMP-7) are about 60% identical to that of BMP-2, and the C-terminal domains of BMP-6 and OP-1 are 87% identical. BMP-6 is likely the human homolog of the murine Vgr-1 (Lyons et al., Proc. Natl. Acad. Sci. U.S.A., 86, pp. 4554-59 (1989)); the two proteins are 92% identical overall at the amino acid sequence level (U. S. Patent No. 5,459,047). BMP-6 is 58% identical to the *Xenopus* Vg-1 product.

### Biochemical Structural and Functional Properties of Bone Morphogenic Proteins

The naturally occurring bone morphogens share substantial amino acid sequence homology in their C-terminal regions (domains). Typically, the above-mentioned naturally occurring osteogenic proteins are translated as a precursor, having an N-terminal signal peptide sequence typically less than about 30 residues, followed by a "pro" domain that is cleaved to yield the mature C-terminal domain of approximately 97-106 amino acids. The signal peptide is cleaved rapidly upon translation, at a cleavage site that can be predicted in a given sequence using the method of Von Heijne Nucleic Acids Research, 14, pp. 4683-4691 (1986). The pro domain typically is about three times larger than the fully processed mature C-terminal domain.

Another characteristic of the BMP protein family members is their apparent ability to dimerize. Several bone-derived osteogenic proteins (OPs) and BMPs are found as homo- and heterodimers in their active forms. The ability of OPs and BMPs to form heterodimers may confer additional or altered morphogenic inductive capabilities on morphogenic proteins. Heterodimers may exhibit qualitatively or quantitatively different binding affinities than homodimers for OP and BMP receptor molecules. Altered binding affinities may in turn lead to differential activation of receptors that mediate different signaling pathways, which may ultimately lead to different biological activities or outcomes. Altered binding affinities could also be manifested in a tissue or cell type-specific manner, thereby inducing only particular progenitor cell types to undergo proliferation and/or differentiation.

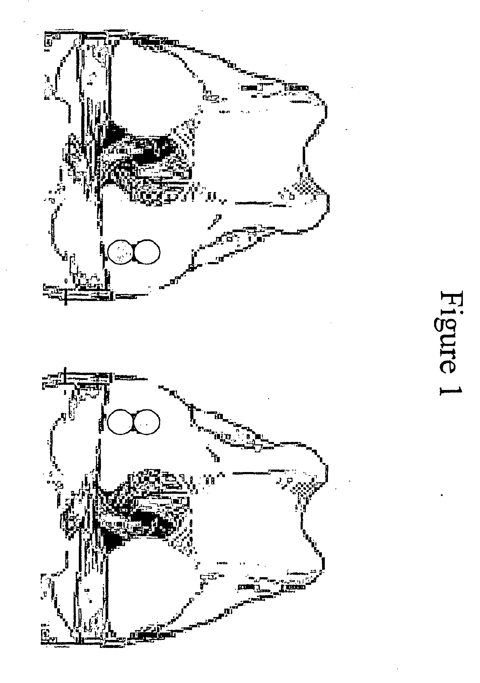

The OP-1 polypeptide shares a defined relationship with a sequence present in osteogenically active human OP-1, SEQ ID NO: 1 (See Fig. 1).

The OP-1 as used herein shares a defined relationship with at least the C-terminal six cysteine domain of human OP-1, residues 335-431 of SEQ ID NO: 1, in particular with at least the C-terminal seven cysteine domain of human OP-1, residues 330-431 of SEQ ID NO: 1.

The osteogenic protein OP-1 has been described (see, *e.g*., Oppermann et al., U. S. Patent No. 5,354,557). Natural-sourced osteogenic protein in its mature, native form is a glycosylated dimer typically having an apparent molecular weight of about 30-36 kDa as determined by SDS-PAGE. When reduced, the 30 kDa protein gives rise to two glycosylated peptide subunits having apparent molecular weights of about 16 kDa and 18 kDa. In the reduced state, the protein has no detectable osteogenic activity. The unglycosylated protein, which also has osteogenic activity, has an apparent molecular weight of about 27 kDa. When reduced, the 27 kDa protein gives rise to two unglycosylated polypeptides, having molecular weights of about 14 kDa to 16 kDa, capable of inducing endochondral bone formation in a mammal. OP-1 may include forms having varying glycosylation patterns, varying N-termini, and active truncated or mutated forms of native protein.

Publications disclosing OP-1 sequences, as well as its chemical and physical properties, include:' (U.S. Patent No. 5,011,691; U.S. Patent No. 5,266,683; Ozkaynak et al., EMBO J., 9, pp. 2085-2093 (1990).

In another embodiment of this invention, the OP-1 protein may be prepared synthetically to induce tissue formation. OP-1 prepared synthetically may be a native, or may be a non-native protein, i.e., one not otherwise found in nature.

In one preferred embodiment of this invention, the OP- comprises a pair of OP-1 subunits disulfide bonded to produce a dimeric species.

In another preferred embodiment of this invention, the OP-1 comprises a pair of OP-1 subunits that produce a dimeric species formed through non-covalent interactions.

Non-covalent interactions include Van der Waals, hydrogen bond, hydrophobic and electrostatic interactions.

As noted above, the-OP-1 useful in the present invention generally is a dimeric protein comprising a folded pair of polypeptides. Such OP-1 is inactive when reduced, but is active as oxidized homodimer.

In some embodiments, the OP-1 is a monomer.

The OP-1 useful in the present invention includes any OP-1, whether isolated from naturally-occurring sources, or produced by recombinant DNA or other synthetic techniques, and includes allelic and phylogenetic counterpart variants of this protein, as well as muteins thereof, and various truncated and fusion constructs. Deletion or addition mutants also are envisioned to be active, including those which may alter the conserved C-terminal six or seven cysteine domain, provided that the alteration does not functionally disrupt the relationship of these cysteines in the folded structure. Accordingly, Such active forms are considered the equivalent of the specifically described OP-1 disclosed herein. The OP-1 may include forms having varying glycosylation patterns, varying N-termini, a family of related proteins having regions of amino acid sequence homology, and active truncated or mutated forms of native or biosynthetic proteins, produced by expression of recombinant DNA in host cells.

The OP-1 contemplated herein can be expressed from intact or truncated cDNA or from synthetic DNAs in prokaryotic or eukaryotic host cells, and purified, cleaved, refolded, and dimerized to form morphogenically active compositions. Currently preferred host cells include, without limitation, prokaryotes including *E*. *coli* or eukaryotes including yeast, or mammalian cells, such as CHO, COS or BSC cells. One of ordinary skill in the art will appreciate that other host cells can be used to advantage. Detailed descriptions of the OP-1 useful in the practice of this invention, including how to make, use and test them for osteogenic activity, are disclosed in numerous publications, including U.S. Patent Nos. 5,266,683 and 5,011,691, as well as in any of the publications recited herein.

Thus, in view of this disclosure and the knowledge available in the art, skilled genetic engineers can isolate genes from cDNA or genomic libraries of various different biological species, which encode appropriate amino acid sequences, or construct DNAs from oligonucleotides, and then can express them in various types of host cells, including both prokaryotes and eukaryotes, to produce large quantities of active proteins capable of stimulating bone and cartilage morphogenesis in a mammal.

### Pharmaceutical Compositions

The pharmaceutical compositions comprising OP-1 may be in a variety of forms. These include, for example, solid, semisolid and liquid dosage forms such as powders, tablets, pills, suppositories, liquid solutions, suspensions, gels, putty, pastes, emulsions and infusible solutions.

The pharmaceutical compositions for use according to this invention is to be administered into the area surrounding the cartilage (e.g., the synovial fluid) in need of repair (i.e. a joint). In other embodiments, the pharmaceutical compositions for use according to this invention may be administered directly into the cartilage tissue (e.g., a meniscus or an 4 intervertebral disc).

The pharmaceutical compositions comprising OP-1 may, for example, be placed into sterile, isotonic formulations with or without cofactors which stimulate uptake or stability. The formulation is preferably liquid, or may be lyophilized powder. For example, the morphogenic protein may be diluted with a formulation buffer. The solution can be lyophilized, stored under refrigeration and reconstituted prior to administration with sterile Water-For-Injection (USP).

The compositions also will preferably include conventional pharmaceutically acceptable carriers well known in the art (see, *e.g.*, Remington's Pharmaceutical Sciences, 16th Ed., Mac Publishing Company (1980)). Such pharmaceutically acceptable carriers may include other medicinal agents, carriers, genetic carriers, adjuvants, excipients, etc., such as human serum albumin or plasma preparations. Preferably, the carrier is isotonic with the blood or synovial fluid of the patient. Examples of such carrier vehicles include water, saline, Ringer's solution, a buffered solution, hyaluronan and dextrose solution. Nonaqueous vehicles such as fixed oils and ethyl oleate are also useful herein. The compositions are preferably in the form of a unit dose and will usually be administered as a dose regimen that depends on the particular tissue treatment.

In some embodiments, the compositions of this invention are sustained release formulations, slow delivery formulations, formulations whereby the morphogenic protein clearance is delayed. There are numerous delivery materials available for preparing these compositions. They include, but are not limited to, microspheres of polylactic/polyglycolic acid polymers, liposomes, Collagen, polyethylene glycol (PEG), hyaluronic acid/fibrin matrices, hyaluronic acid, fibrin, chitosan, gelatin, SABER™ System (sucrose acetate isobutyrate (SAIB)), DURIN™ (biodegradabale polymer for drug loaded implants), MICRODUR™ (biodegradable polymers/microencapsulation) and DUROS™ (mini-osmotic pump). In some embodiments, the morphogenic protein is covalently linked to the delivery material.

The compositions for use according to this invention contain OP-1 dispersed in a biocompatible carrier material that functions as a suitable delivery system for the compounds. Suitable examples of sustained release carriers include semipermeable polymer matrices. Implantable or microcapsular sustained release matrices include polylactides (U.S. Patent No. 3,773,319; EP 58,481), copolymers of L-giutamic acid and gamma-ethyl-L-glutamate (Sidman et al., Biopolymers, 22, pp. 547-56 (1985)); poly(2-hydroxyethyl-methacrylate), ethylene vinyl acetate (Langer et al., J. Biomed. Mater. Res., 15, pp. 167-277 (1981); Langer, Chem. Tech., 12, pp. 98-105 (1982)) or poly-D-(-)-3hydroxybutyric acid (EP 133,988), polylactic acid, poly glycolic acid or polymers of the above,

The pharmaceutical compositions for use according to this invention may also be administered using, for example, microspheres, liposomes, other microparticulate delivery systems or sustained release formulations placed in, near, or otherwise in communication with affected tissues, the fluids bathing those tissues (e.g., synovial fluid) or bloodstream bathing those tissues.

Liposomes containing OP-1 can be prepared by well-known methods (See, e.g. DE 3,218,121; Epstein et al., Proc. Natl. Acad. Sci. U.S.A., 82, pp. 3688-92 (1985); Hwang et al., Proc. Natl. Acad. Sci. U.S.A., 77, pp. 4030-34 (1980); U.S. Patent Nos. 4,485,045 and 4,544,545). Ordinarily the liposomes are of the small (about 200-800 Angstroms) unilamellar type in which the lipid content is greater than about 30 mol.% cholesterol. The proportion ofcholesterol is selected to control the optimal rate of morphogenic protein release.

The OP-1 used in of this invention may also be attached to liposomes.

Attachment of OP-1 to liposomes may be accomplished by any known cross-linking agent such as heterobifunctional crosslinking agents that have been widely used to couple toxins or chemotherapeutic agents to antibodies for targeted delivery. Conjugation to liposomes can also be accomplished using the carbohydrate-directed cross-linking reagent 4-(4-maleimidophenyl) butyric acid hydrazide (MPBH) (Duzgunes et al., J. Cell. Biochem. Abst. Suppl. 16E 77 (1992)).

One skilled in the art may create a biocompatible, and or biodegradable formulation of choice to promote tissue induction.

A successful carrier for OP-1 should perform several important functions. It should act as a slow release delivery system of OP-1 or delay clearance of the morphogenic protein, and protect the morphogenic protein from non-specific proteolysis.

In addition, selected materials must be biocompatible *in vivo* and preferably biodegradable. Polylactic acid (PLA), polyglycolic acid (PGA), and various combinations have different dissolution rates *in vivo.*

The carrier may also take the form of a hydrogel. When the carrier material comprises a hydrogel, it refers to a three dimensional network of crosslinked hydrophilic polymers in the form of a gel substantially composed of water, preferably but not limited to gels being greater than 90% water. Hydrogel can carry a net positive or net negative charge, or may be neutral. A typical net negative charged hydrogel is alginate. Hydrogels carrying a net positive charge may be typified by extracellular matrix components such as collagen and laminin. Examples of commercially available extracellular matrix components include Matrigel™ and Vitrogen™. An example of a net neutral hydrogel is highly crosslinked polyethylene oxide, or polyvinyalcohol.

Dosage levels of between about 1 µg and about 1000 µg per day, preferably between 3 µg and 50 µg per day of OP-1 are useful in cartilage repair and regeneration. As the skilled artisan will appreciate, lower or higher doses than those recited may be required. Specific dosage and treatment regimens for any particular patient will depend upon a variety of factors, including the activity of the specific morphogenic protein employed, the age, body weight, general health status, sex, diet, time of administration, rate of excretion, drug combination, the severity of the tissue damage and the judgment of the treating physician.

### EXAMPLE 1: Dog Model Repair of Osteochondral Defects

12 adult male bred for purpose dogs will undergo surgery. Both hindlimbs will be prepped and draped in sterile fashion. A medial parapatellar incision approximately four centimeters in length will be made. The patella will be retracted laterally to expose the femoral condyle. In the right medial condyle, a 5.0 mm diameter defect extending through the cartilage layer and penetrating the subchondral bone to a depth of 6 nun will be created in the central load bearing region of the femoral condyle with a specially designed or modified 5.0 mm drill bit. The animals will be divided into two groups of 6 animals each. In the first group, after copious irrigation with saline to remove debris and spilled marrow cells, the appropriate time release OP-1 will be applied to the synovial fluid surrounding the defect. In the first group of 6 animals, the right defects will receive the time release OP-1. The left limb of all animals will serve as a control receiving control beads (0% OP-1).

The second group of 6 animals will receive no OP-I treatment at the time of surgery. At 3 days post surgery, the appropriate time release OP-1 formulation will be injected into the synovial fluid surrounding the joint with the defect. In 6 animals, time release OP-1 will be injected into the synovial fluid around the right defect. The left limb of all animals will serve as a control receiving control beads (0% OP-1).

The animals will be sacrificed at 16 weeks post-surgery. At sacrifice, the distal femurs will be retrieved *en bloc* and the defect sites will be evaluated histologically and grossly based on the scheme of Moran et al (J. Bone Joint Surg. 74B:659-667, 1992) that has been used in previous investigations.

Radiographs of the hindlimbs will be obtained preoperatively, immediately postoperative, and at postoperative week 16. The preoperative radiographs will be used to assure that no pre-existing abnormalities are present and to verify skeletal maturity. Post-operative radiographs will be used to assess defect placement. Sacrifice radiographs will be used to assess the rate of healing and restoration of the subchondral bone and the articulating surface. Radiographs will be obtained within one week of the evaluation date.

Gross pathological examination of the carcasses will be conducted immediately after sacrifice. The distal femurs will be immediately harvested *en bloc* and stored in saline soaked towels and placed in labeled plastic bags. High power photographs of the defect sites will be taken and carefully labeled.

Soft tissues will be meticulously dissected away from the defect site and the proximal end of the femur will be removed. On a water cooled diamond cut saw each defect site will be isolated for histologic evaluation.

Specimens will be fixed by immersion in 4% paraformaldehyde solution and prepared for decalcified histologic processing. Three sections from three levels will be cut from each block. Levels 1 and 3 will be closest to the defect perimeter. Level 2 will be located at the defect center. Three sections from each level will be stained with toluidine blue and Safranin O and fast green. Sections will be graded based on the scheme of Moran et al. (J. Bone Joint Surg. 74B:659-667, 1992).

It is expected that OP-1 treated animals will exhibit improved repair of the osteochondral defects when compared to control animals.

### EXAMPLE 2: Sheep Model of Regeneration of Chondral Defects By Intra-articular Administration of OP-1 in Time-Release Microspheres

18 adult bred for purpose sheep will undergo surgery. With a specially designed instrument, a 10mm chondral defect will be created in the left hindlimb knee of 18 sheep on the weight bearing condyle surface, 2mm deep up to the calcified layer (exposition of blood will be pronounced as a failure). The right knees of all animals will remain untouched to serve as a control.

Group 1 (6 animals): At postoperative day 3, the left knee of each animal will receive an intra-articular injection of a 250µl suspension containing 57mg of control 0.3% microspheres without OP-1.

Group 2 (6 animals): At postoperative day 3, the left knee of each animal will receive an intra-articular injection of a 250µl suspension containing 57mg of 0.3% microspheres containing 170pg of OP-1.

Group 3 (6 animals): At postoperative day 3 and at postoperative week 6, the left knee of each animal will receive an intra-articular injection of a 250µl suspension containing 57mg of 0.3% microspheres containing 170µg of OP-1.

Arthroscopic evaluation of the knees will be performed at postoperative weeks 3 and 6 on all the animals. NMR/MRI scans will be performed at postoperative week 3 and 6. Mechanical testing of the knees will also be performed periodically.

All animals will be sacrificed at 3 months postoperative. After sacrifice, histology, histomorphometry, immunostaining, and in situ hybridization for specific articular chondrocyte markers will be performed. It is expected that OP-1 treated knees will exhibit improved regeneration when compared to control knees.

### EXAMPLE 3: Sheep Model for Prevention of Osteoarthritis

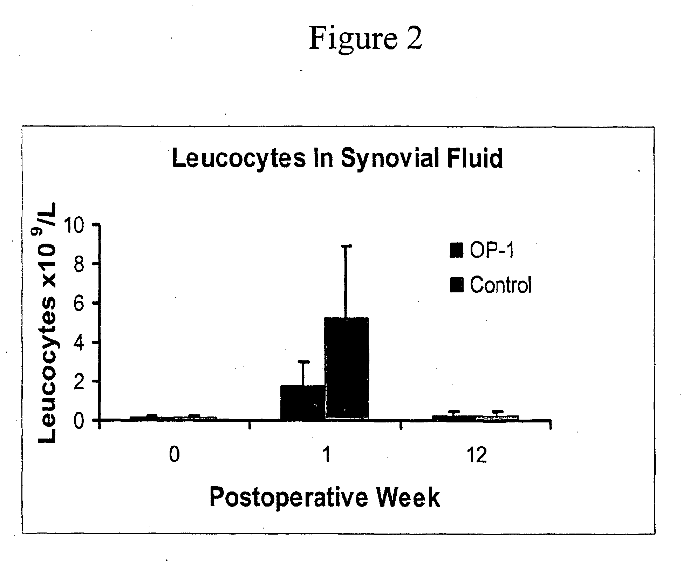

Sheep are used as a model for osteoarthritis because it has been demonstrated that progressive osteoarthritis occurs in these animals after a single injury impact. Twelve adult female crossbred sheep that are acclimatized for 14 days were used in this study. All sheep received general anesthesia and using aseptic techniques, a 3 cm arthrotomy was used to allow access to both femorotibial joints. A spring loaded mechanical device was used to create bilateral impact injuries to the weight bearing region of the median femoral condyle (30 Mpa, 6mm diameter x 2) (see figure 1). After a routine closure of these incisions, the sheep received an intra-articular injection in each knee of OP-1 in a vehicle of collagen and carboxymethylcellulose (OP-1 Implant, 340µg) or vehicle alone. Two experimental groups (N=6) were used. Group A received 0.3 ml of OP-1 + collagen + carboxymethylcellulose intra-articularly in one knee at the time of surgery (day 0) and one week later (day 7). Day 0 injections were administered immediately after the surgical incision is closed. Group B received OP-1 in one knee on day 0, 7, 14, 21, 28, and 35. Synovial fluid was aspirated before injection of the OP-1 and vehicle to allow measurement of leukocyte numbers and total protein as indicators of inflammation. OP-1 treatment significantly reduced leucocytes in synovial fluid 1 week postoperatively (p<.003, paired T test) but not total protein concentration (see figure 2).

The sheep were sacrificed 12 weeks postoperatively for detailed assessment (paravital staining, TUNEL staining, histopathology, cartilage, sulfated GAG analysis, biomechanical indentation testing) of the articular tissues. Abnormal cartilage (India ink uptake) was significantly different between groups ((p<.03) because lesions in OP-1 knees were often limited to reduced sheen/reflectivity whereas control joints had areas of fibrillation or erosion (see table 1).

**Table 1**

| | **Abnormal Cartilage %¹** | |
|---|---|---|
| **Animal** | **Vehicle** | **OP-1** |
| 28 | 20 | 5 |
| 29 | 40 | 20 |
| 30 | 60 | 0 |
| 31 | 50 | 20 |
| 32 | 70 | 10 |
| 33 | 25 | 10 |

| | | |
|---|---|---|
| ¹From India ink uptake on joint surface, digital photography, scaled area measurements using Northern Eclipse™ morphometry software. | | |

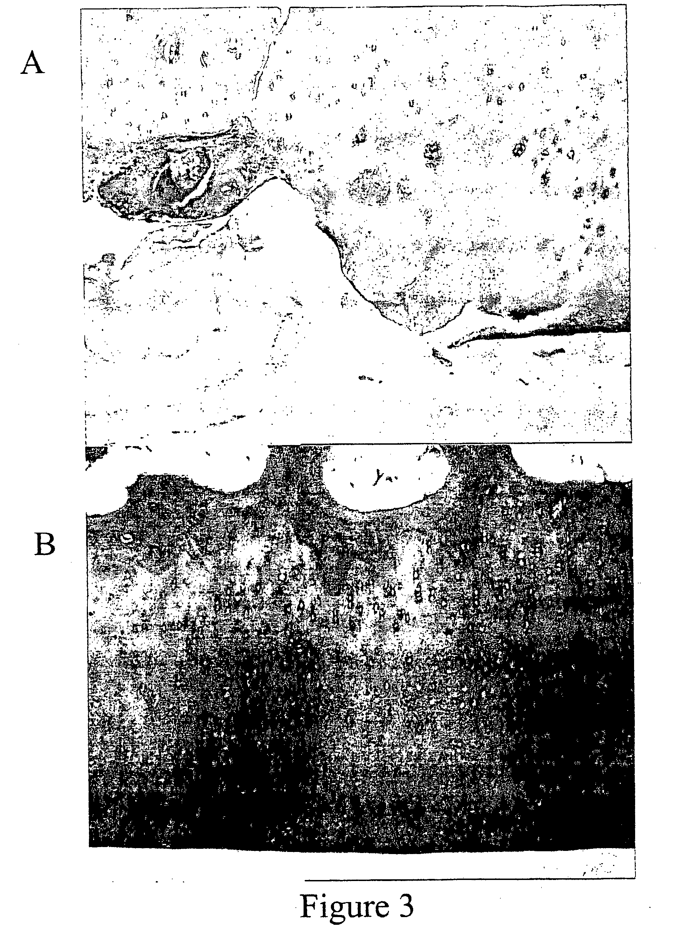

Histological sections showed chondrocyte clusters, acellular matrix and cartilage loss in vehicle treated joints (figure 3A), whereas lesions in OP-1 treated joints (figure 3B) were superficial zone chondrocyte loss and/or small fissures. Mankin histology scoring was not significantly different (p<.06, Wilcoxon Signed Rank Test); but the OARSI scoring system that is sensitive to the size of the lesion proved valuable (p<.03) (see table 2) (van der Sluijs J. et al., The reliability of the Mankin score for osteoarthrits. Ortho Res 1992, 10:58-61). Table 2

| | **Modified Mankin Score¹** | | **OARSI Score²** | |
|---|---|---|---|---|
| **Animal** | **Vehicle** | **OP-1** | **Vehicle** | **OP-1** |
| 28 | 4 | 2 | 6 | 2 |
| 29 | 4 | 2 | 8 | 1.5 |
| 30 | 4 | 2 | 8 | 1 |
| 31 | 5 | 1 | 12 | 5 |
| 32 | 4 | 3 | 13.5 | 4 |
| 33 | 6 | 3 | 10 | 4 |

| | | | | |
|---|---|---|---|---|
| ¹Modified Mankin score is 0-13 where 0 is normal cartilage. ²Osteoarthritis Research Society International Score calculation=lesion severity x area with a maximum of 24 for a single lesion. | | | | |

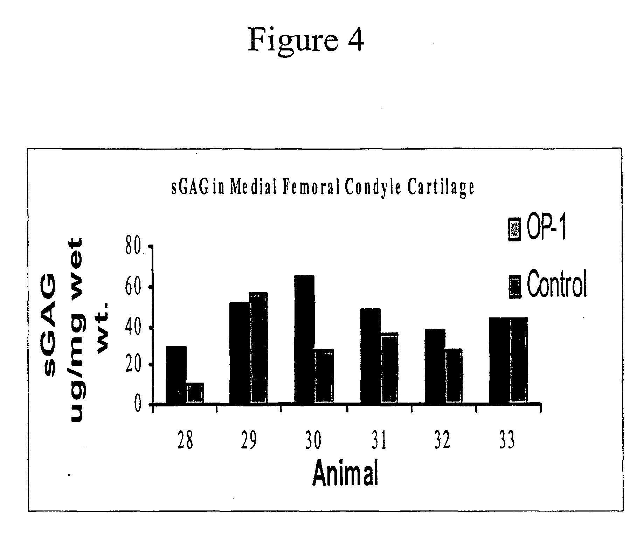

Sulfated glycosaminoglycan concentrations were higher in the OP-1 treated group with a strong trend toward statistical significance (p<.06) (see figure 4).

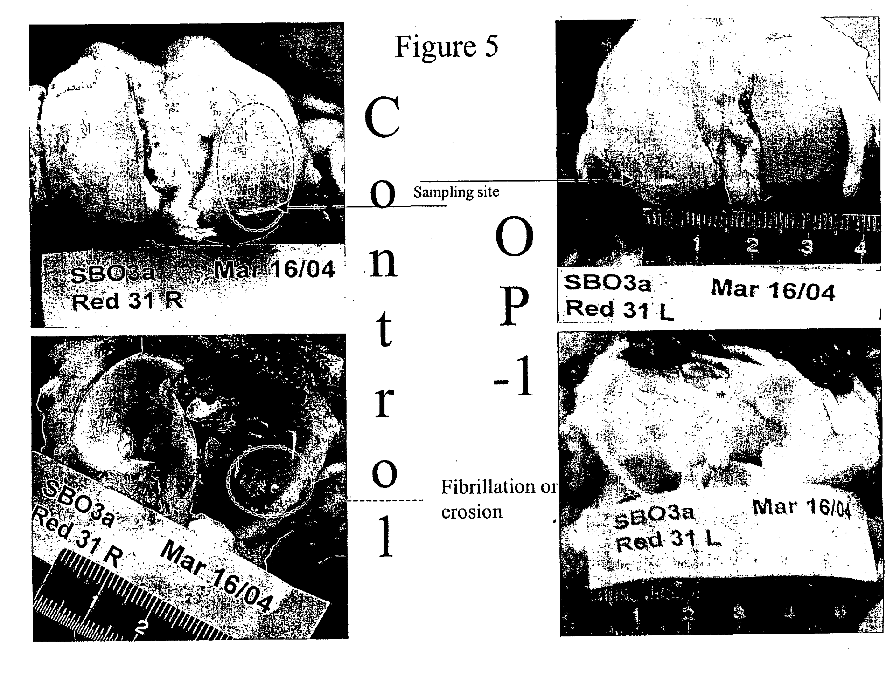

The collagen/CMC alone group resulted in fibrillations and erosion of the surface, whereas the OP-1 group shows little or no sign of damage (see figure 5). The OP-1 treated joints look healthier and shinier than the controls.

These experiments demonstrate marked improvement, if not complete protection with two injections of OP-1. Small lesions may persist in the face of therapy because 30 MPa impact injuries partial thickness defects may occur that are unlikely to repair completely. OP-1 was able to suppress the centrifugal extension of degenerative changes over the femoral condyle, whereas vehicle treated joints developed a unicompartmental osteoarthritis. The mechanism by which OP-1 exerts this effect maybe through its anabolic properties by affecting repair. However, little repair tissue was present at the impacted sites so another mechanism that promotes survival of injured chondrocytes may be operative. These observations indicate that OP-1 may be useful for other applications such as tissue engineering and cell based therapies where injury might occur when cells are harvested or handled.

### EXAMPLE 4: Sheep Model for Therapeutic Effect of OP-1 after Intra-Articular Injection

This study will use N=12 adult female 1.5-2.5 year old crossbred sheep that are acclimatized for 14 days and pass a health status assessment before entry into the study. Under general anesthesia and using aseptic technique, all sheep will receive standardized 30 MPa impact injuries to both (left and right) medial femoral condyles by a 3 cm minimally invasive arthrotomy. Three weeks postoperatively the sheep will be sedated with diazepam (10mg/kg) and ketamine (3-5mg/kg) to allow aseptic preparation of knee for synoviocentesis and injection of test article, placebo or physiologic saline into the medial femorotibial joint according the to Table 3.

**Table 3**

| | | **Week** | | **0** | **3** | **4** | **8** | **12** | **16** |
|---|---|---|---|---|---|---|---|---|---|
| **Dose** | **Group** | **# animals** | **# Knees** | **Surgery** | **intra-articular injection** | | | | |
| two doses 3 & 4 weeks post injury | Test-L | 9 | 9 | Impact Injury | OP-1/P | OP-1/P | | | sacrifice |
| | Placebo-R | | 9 | | Placebo | Placebo | | | |
| saline controls | Saline control-R | 3 | 6 | Impact Injury | Saline | Saline | | | sacrifice |
| | Saline control-L | | | | None | None | | | |
| **Total animals in study** | | **12** | | | | | | | |
| Synovial Fluid Aspirate | | | | x | x | x | x | x | x |

All sheep will receive bilateral medial femoral condyle injuries. In the first group of nine sheep, one knee will receive the test article and the contralateral knee will receive a placebo consisting of the vehicle alone. Knee treatments will be allocated by a complete block design. A second group of three sheep will receive physiologic saline USP as a control for the effect of the placebo.

The study will follow the following procedure set forth in Table 4:

**Table 4**

| | |
|---|---|
| Day -14 to day -1 | Preconditioning, health maintenance program, foot trimming, Q-fever test |
| Week 0 | Surgery and impact injury to both knees of sheep. |
| Week 3, 4 | Synovial fluid collection. |
| | Synovial fluid harvested and OP-1 and placebo injected into respective joints. |
| Week 8, 12 | Synovial fluid harvested using aseptic technique and sedation. Freeze 2 aliquots synovial fluid (200 uL each) and process one fresh EDTA aliquot for total leukocyte count, differential counts and total protein determination. |
| Week 16 | Sacrifice all sheep. Harvest synovial fluid and tissues for detailed assessments |

### EXAMPLE 5: Guinea Pig and Rabbit Models of Osteoarthritis

The Hartley guinea pig (spontaneous) and rabbit ACL-resection (induced) osteoarthritis models were utilized. Fourteen guinea pigs of either 3, 6 or 9 months of age were injected in the right knee with a phosphate buffered saline (PBS) solution containing 50 µg rhOP-1 at 3-week intervals for a period of 12 weeks. The left knee served as an untreated control.

In ten New Zealand White rabbits, the left ACL was resected and received either an injection into the joint of 100 µg rhOP-1 in a PBS solution or a control solution at 3-week intervals during a 12-week evaluation period. The right knee served as a non ACL- resected nontreated control in all animals.

All animals in both models were evaluated for gross appearance and histologic evidence of arthritic changes using a modified Mankin scale to grade the severity of degeneration. The untreated guinea pig knees developed a progression of arthritic changes from 3 to 6, 6 to 9 and 9 to 12 months of age with severe degeneration apparent grossly and histologically at 12 months of age. The OP-1 treatment had the most profound effect in preventing degeneration in the guinea pig at the early time periods. Gross and histologic degeneration in the knee at 9 months of age in rhOP-1 treated animals were similar to untreated animals at 6 months of age. At 12 months of age, the severity of degenerative changes was comparable. In the rabbit ACL-resected model OP-1 treatment showed slight improvement in the severity of degeneration in treated sites at the 12 week evaluation period. These results demonstrate that OP-1 has some beneficial effects in preventing or slowing early stage arthiritic changes.

### EXAMPLE 6: Sheep Model of Meniscus Healing

A hole (6mm diameter) and a longitudinal tear (2cm long) sutured by non-resorbable thread were created in each medial meniscus of both knees of sheep. There were two treatment groups: OP-1 putty (3.5 mg OP-1/gram of Bovine type 1 collagen with carboxymethylcellulose) and a control group with no treatment other than the surgically created defect. The OP-1 treated animals received 0.3 mls (350mcg) injected into the joint space just prior to closing the incision and then injected into the joint space 7 days after surgery.

6, 12 and 26 weeks after treatment, the animals will be euthanized. After euthanasia, the meniscus will be removed and cut in two parts, the anterior, longitudinal sutured tear and the posterior, with the hole. The sections will be stained with Masson's Trichrome and safranin O. Immunohistochemistry of the meniscus may also be performed using specific antibodies to detect collagen I, II, VI, S100, proteases MMP1.

A section of meniscus will be separated, embedded in OCT and frozen in liquid nitrogen. Sections obtained with a cryostat will be collected, homogenized and RNA prepared using Trizol reagent. RT-PCR will be performed to study gene expression of various markers including type I, type II, type II collagen and aggrecan as markers for extracellular matrix, TGF-β and IGF-2 as growth factors, MMP-1, MMP-3 and TIMP-1 as matrix degrading enzymes, and finally cyclin A, Bcl-2, BAX and caspase 3 as markers for proliferating and apoptotic state of cells. Other joint tissue will also be inspected and compared to controls for any gross differences which may be caused by OP-1.

Preliminary results on effect of OP-1 putty in holes in the avascular area of the meniscus indicate that in all the menisci with hole defects a positive effect was noted after treatment with OP-1 putty. The putty remained for the first six weeks, and later was reabsorbed and disappeared. Notably, there was considerable penetration of cells from the surface of the meniscus to the inside of the holes, which were mainly filled with fibrous tissue from the eighth week onwards.

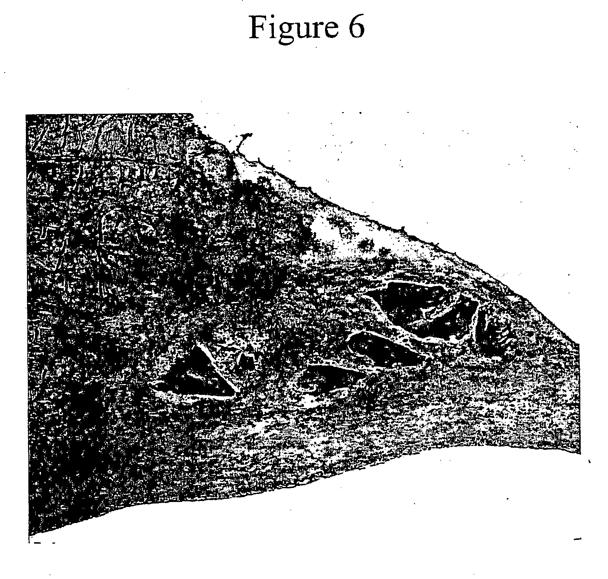

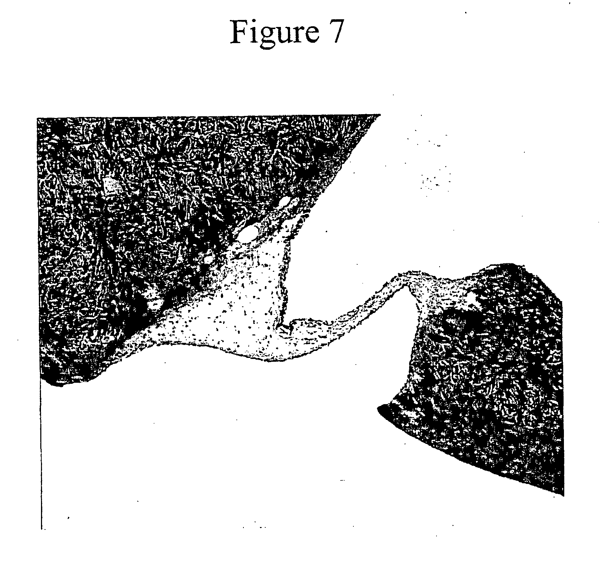

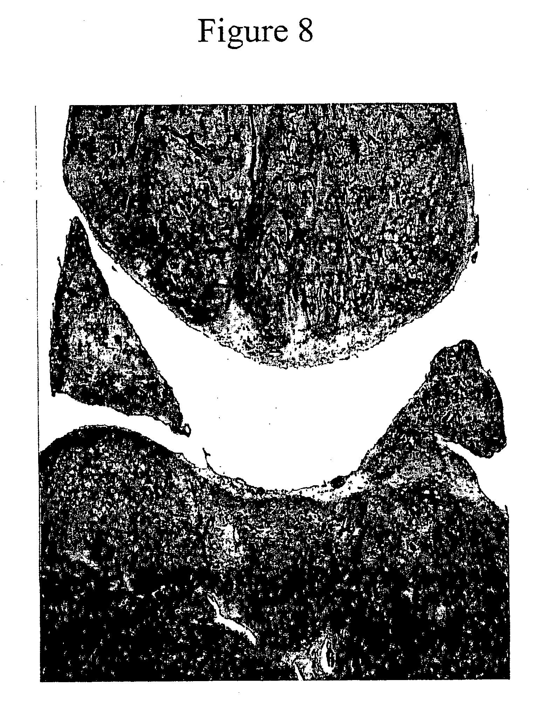

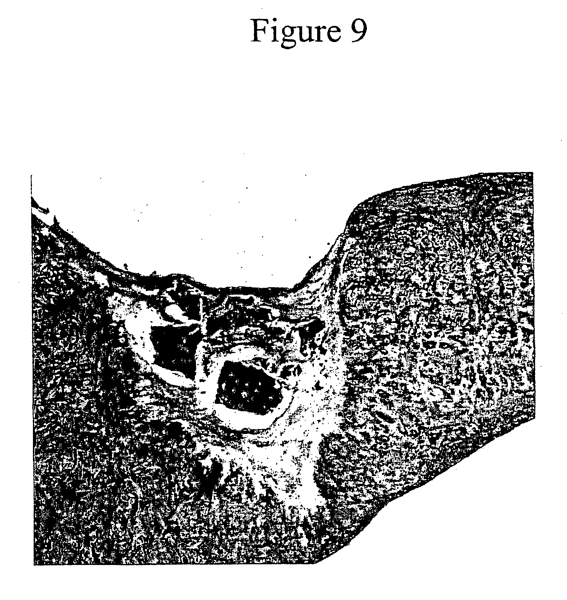

At 6 weeks, most of the control animals had little material filling the defects, and the material present was fibrous and whispy. In the OP- treated defects, there was more tissue present along with large particulate collagen. Cellular response appeared to be higher in the OF-1 group (see figures 6 and 7). By 12 weeks, most of the control defects remained empty. Little cellular activity was seen along the periphery of the defect. The OP-1 treated defects still contained collagen particles, but there appeared to be an increase in cellularity surrounding the defect, and some progression to new tissue formation (see figure 8). After 25 weeks, fibrous bridging was seen in a few of the control animals, but most of this was tenuous in nature. The collagen particles disappeared from the defects in the OP-1 group and were replaced predominantly with fibrous tissue: Remodeling appeared to remain active (see figure 9).

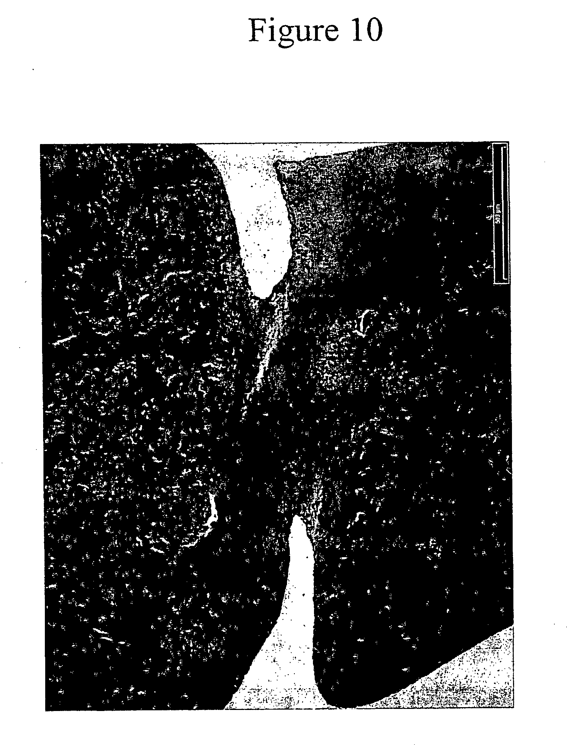

Preliminary results on the effect of OP-1 putty on the repair of menisci with longitudinal lesions were not conclusive. Only small differences were observed from the lesions treated with OP-1 when compared to the control group. This could be due to the fact that suturing does not provide adequate fixation, and that the protein does not integrate well because of the suture. In a few OP-1 treated animals bridging of the defect could be observed (see figure 10).

### REFERENCE EXAMPLE 7: Sheep Model of Disc Repair and Regeneration

Experimental induction of controlled outer annular defects in sheep initiates a sequence of events which closely reproduces, pathologically and biochemically, the evolution of disc degeneration in man. Compositional changes include an alteration in the amount of, and the types of collagens synthesized by cells of the lesion site Kaapa et al 1994a, b, 1995 Kaapa E. et al. (1995) Collagen synthesis and types I, III, IV, and VI collagens in an animal model of disc degeneration, Spine 20, 59-67; Kaapa E et al., (1994) Collagens in the injured porcine intervertebral disc, J. Orthop. Res. 12. 93-102; and Kaapa E et al., (1994) Proteoglycan chemistry in experimentally injured porcine intervertebral disk, J. Spin. Dis. 7, 296-306) loss of large high buoyant density aggrecan type proteoglycans and an elevation in levels of the small DS substituted proteoglycans decorin and biglycan in the injured disc (Melrose J. et al, (1992) A longitudinal study of the matrix changes induced in the intervertebral disc by surgical damage to the annulus fibrosus, J Orthop Res 10:665-676; Melrose J. et al., (1997) Topographical variation in the catabolism of aggrecan in an ovine annular lesion model of experimental disc degeneration J Spinal Disord 10:55-67; and Melrose J. et al., (1997) Elevated synthesis of biglycan and decorin in an ovine annular lesion model of experimental disc degeneration, Eur Spine J 6:376-84). Changes in the vascular supply to the cartilaginous end plate (CEP) (Moore RJ et al., (1992) Changes in endplate vascularity after an outer anulus tear in the sheep, Spine 17:874-878) and remodelling of vertebral bone adjacent to experimental annular defects (Moore RJ, et al. (1996) Remodeling of vertebral bone after outer anular injury in sheep, Spine 21:936-940.), changes in the biomechanical competence of "repaired" lesion affected discs (Latham JM et al., (1994) Mechanical consequences of annular tears and subsequent intervertebral disc degeneration, J Clin Biomech 9:211-9), and osteoarthritic changes in spinal facet joints (Moore RJ et al., (1999) Osteoarthrosis of the facet joints resulting from anular rim lesions in sheep lumbar discs, Spine, 24:519-525) as a consequence of disc degeneration have also been noted.

### A. The ovine annular lesion model

The sheep will be fasted for 24 h prior to surgery and anaesthesia will be induced with an intravenous injection of 1g thiopentone. A lateral plain X-ray film will be taken to verify normal lumbar spine anatomy. General anaesthesia will be maintained after endotracheal intubation by 2.5% halothane and monitored by pulse oximetry and end tidal CO₂ measurement. The left flank from the ribs to the iliac crest will be prepared for sterile surgery. The sheep will receive an intramuscular injection of 1200 mg penicillin. A skin incision will be made on the left side immediately anterior to the transverse processes of the spine and the lumbar spine will be exposed by blunt dissection using an anterior muscle-splitting technique. The vascular and neural anatomy will be respected and bleeding will be controlled by direct pressure or electrocautery as required.

A total of twelve two year old sheep will receive controlled annular lesions in their L1-L2, L3-L4 and L5-L6 discs by incision through the left anterolateral annulus fibrosus parallel and adjacent to the cranial endplate using a #11 scalpel blade to create a lesion measuring 4 mm long x 5 mm deep. The intervening lumbar discs (L2-L3, L4-L5) will not be incised.

The incised discs will receive one of 3 therapies, (I) no treatment, (II) lactose solution or (III) lactose containing OP-1. In all sheep the L3-L4 disc will receive an annular lesion with no treatment. In 4 sheep the L1-L2 discs will be treated with lactose solution only and the L5-L6 disc will be treated with lactose plus OP-1. In the remaining 4 sheep the treatments in the L1-L2 and L5-L6 discs will be reversed to avoid any potential outcome bias associated with spinal level. A non-operated disc must remain between treated discs to allow for adequate anchorage of FSUs in subsequent mechanical testing (see below). A wire suture will be used to identify the craniad operated level for later identification purposes both in X-rays and for morphological identification. Three additional non-operated animals will also be used as controls for the biomechanical study.

Degeneration following annular incision is well established in the sheep (Osti OL et al., (1990) Volvo Award for Basic Science, Annulus tears and intervertebral disc degeneration. An experimental study using an animal model, Spine 15:762-7) and can be expected to show the earliest radiographic and histochemical evidence after 12 weeks. Three months after induction of the annular lesions the sheep will be killed by intravenous injection of 6.5 g sodium pentobarbitone and the lumbar spines will be radiographed to evaluate disc calcification, excised and processed for biomechanical (n=8) and histochemical (n=4) analyses, and, after the biomechanical testing the same discs will be zonally dissected for compositional analyses.

### B. Compositional analysis of disc tissues

Intervertebral disc tissues will be zonally dissected into annular quadrants and nucleus pulposus as depicted in Figure 11.

### C. Determination of Proteoglycan and Collagen Contents of Disc Tissues

Samples of annulus fibrosus and nucleus pulposus will be finely diced over ice and representative portions of each tissue zone of known wet weight will be freeze dried to constant weight. The difference between the starting and final weights of the tissues will provide their water contents. Triplicate portions (1-2 mg) of the dried tissues will be hydrolyzed in 6M HCl at 110°C for 16 h and aliquots of the neutralized digests assayed for hydroxyproline as a measure of the tissue collagen content (Melrose J et al., (1992) A longitudinal study of the matrix changes induced in the intervertebral disc by surgical damage to the annulus fibrosus, J Orthop Res 10:665-676; Melrose J et al., (1994a) Proteoglycan heterogeneity in the normal-adult ovine intervertebral disc, Matrix 14:61-75; Melrose J et al., (1994b) Variation in the composition of the ovine intervertebral disc with spinal level and in its constituent proteoglycans, Vet Comp Orthop Traum 7:70-76; Melrose J et al., (1991) The influence of scoliosis and ageing on proteoglycan heterogeneity in the human intervertebral disc J Orthop Res 9:68-77; and Melrose J et al., (1996) Intervertebral disc reconstitution after chemonucleolysis with chymopapain is dependent on dosage: an experimental study in beagle dogs Spine 21:9-17). Triplicate portions of dried tissues (∼2 mg) will also be digested with papain and aliquots of the solubilized tissue assayed for sulphated glycosaminoglycan using the metachromatic dye 1, 9-dimethylmethylene blue as a measure of tissue proteoglycan (see Melrose et al 1991, 1992, 1994, 1996, supra).

### D. Histochemical and Immunohistochemical Analyses

Spinal motion segments that are designated for histochemical analysis will be isolated by cutting through the cranial and caudal vertebral bodies close to the cartilaginous endplates using a bone saw. Entire disc specimens including the adjacent vertebral body segments will be fixed en bloc in either 10% neutral buffered formalin or Histochoice^{®} for 56 h and decalcified in several changes of 10% formic acid in 5% NBF for 2 weeks with constant agitation until complete decalcification is confirmed using a Faxitron HP43855A X-ray cabinet (Hewlett Packard, McMinnville, USA).

Sagittal slices (5 mm thick) of the decalcified disc-vertebral body specimens will be dehydrated through graded ethanol solutions by standard histological methods and embedded in paraffin wax. Paraffin sections 4 µm thick will be prepared for histochemical staining and mounted on Superfrost Plus glass microscope slides (Menzel-Glaser) and dried at 85°C for 30 min then at 55°C overnight. The sections will be deparaffinized in xylene (4 changes x 2 min) and rehydrated through graded ethanol washes (100-70% v/v) to water.

Three sections from all blocks will be stained with haematoxylin and eosin. These sections will be coded and examined by an independent histopathologist who will compare the histological characteristics of those levels that received annular incision only with those that were incised and received rhOP-1. A four-point semi-quantitative grading system will be used to assess the microscopic features. Collagen architecture will also be examined in sections stained with Masson's trichrome and picro-sirius red using polarized light microscopy.

The immunohistochemistry procedures will be performed using a Sequenza cassette and disposable Coverplate immunostaining system as described earlier (Melrose J et al., (2002) Perlecan, the Multi-domain Proteoglycan of Basement Membrane is also a Prominent Pericellular Component of Hypertrophic Chondrocytes of Ovine Vertebral Growth Plate and Cartilaginous End Plate Cartilage, Histochem. Cell Biol. 118, 269-280; Melrose J et al., (2002) Increased nerve and blood-vessel in-growth associated with proteoglycan depletion in an ovine annular lesion model of experimental disc degeneration, Spine 27, 1278-85; Melrose J et al., (2002) Comparison of the morphology and growth characteristics of intervertebral disc cells, synovial fibroblasts and articular chondrocytes in monolayer and alginate bead cultures, Eur. Spine J. 12, 57-65; Melrose J et al. (2001) Differential expression of proteoglycan epitopes and growth characteristics of ovine intervertebral disc cells grown in alginate beads, Cells Tissues Organs 168:137-146; Melrose J et al., (2003) Perlecan, the multi domain HS-proteoglycan of basement membranes is a prominent extracellular and pericellular component of the cartilaginous vertebral body rudiments, vertebral growth plates and intervertebral discs of the developing human spinal column, J Histochem Cytochem 51:1331-1341; Melrose J et al., (2000) Differential Expression of Proteoglycan epitopes by ovine intervertebral disc cells grown in alginate bead culture, J. Anat. 197:189-198; Melrose J et al., (2002) Spatial and Temporal Localisation of Transforming Growth Factor-β, Fibroblast Growth Factor-2, Osteonectin and Identification of Cells Expressing α-Smooth Muscle Actin in the Injured Annulus Fibrosus: Implications for Extracellular Matrix Repair, Spine 27:1756-1764; and Knox S et al., (2002) Not all perlecans are created equal : interactions with fibroblast growth factor-2 (FGF-2) and FGF receptors, J. Biol. Chem. 277:14657-14665). Endogenous peroxidase activity will be initially blocked by incubating the tissue sections with 3% H₂O₂. This will be followed by pre-digestion of the tissue sections with combinations of chondroitinase ABC (0.25 U/ml) in 20 mM Tris-acetate buffer pH 8.0 for 1 h at 37°C, bovine testicular hyaluronidase 1000 U/ml for 1 h at 37°C in phosphate buffer pH 5.0, followed by three washes in 20 mM Tris-HCl pH 7.2 0.5M NaCl (TBS) or proteinase-K (DAKO S3020) for 6 min at room temperature to expose antigenic epitopes. The tissues will then be blocked for 1 h in 20% normal swine serum and be probed with a number of primary antibodies to large and small proteoglycans and collagens (Table 5). Negative control sections will also be processed either omitting primary antibody or substituting an irrelevant isotype matched primary antibody for the authentic primary antibody of interest. Horseradish peroxidase or alkaline phosphatase conjugated secondary antibodies will be used for detection using 0.05% 3, 3'-diaminobenzidene dihydrochloride and 0.03% H₂O₂ in TBS or Nova RED substrates. The stained slides will be examined by bright-field microscopy and photographed using a Leica MPS 60 photomicroscope digital camera system.

**Table 5**

| Primary antibodies to proteoglycan and collagen core protein epitopes | |
|---|---|
| **Primary Antibody epitope** | **Clone (isotype)** |
| **Large Proteoglycans** | |
| Aggrecan | AD 11-2A9 (IgG) |
| Perlecan | A76 (IgG₁) |
| Versican | A1S1D1D1 (IgG) |

| **Small proteoglycans** | |
|---|---|
| Decorin | 6-B-6 (IgG) |
| Biglycan | LF-96 (rabbit IgG) |
| Fibromodulin | Rabbit polyclonal |

| **Collagen** | |
|---|---|
| Type I | 18H5 (IgG₁) |
| Type II | II-4CII (IgG₁) |
| Type IV | CIV-22 (IgG₁) |
| Type VI | Rabbit polyclonal |
| Type X | Mouse polyclonal |

### E. Biomechanical assessment of spinal motion segments

Non-destructive biomechanical range of motion (ROM) analysis will be conducted on each functional spinal unit (FSU) in various planes of motion (flexion-extension, lateral bending, compression and torsion). Each FSU comprises two adjacent vertebrae, the intervening disc and associated ligaments.

A specially designed jig, based on that developed by Callaghan and McGill, allows pure torsion and bending moments to be applied to each FSU while maintaining a constant axial load. This combined loading is a close simulation of the physiological loads experienced by the spine in-vivo.

Four FSUs will be tested: non-operated control levels; levels that were incised; levels that were incised and treated with OP-1 and carrier and levels that were incised and treated with carrier alone. Each FSU will be mounted in two aluminum alloy cups and secured with cold cure dental cement. Care will be taken to ensure that the intervertebral disc is aligned with the cups. Prior to the commencement of testing each FSU will be preloaded to a stress of 0.5 MPa until a reproducible state of hydration is achieved. This is used as the baseline prior to each test. The preload stress of 0.5 MPa simulates relaxed standing and is based on in-vivo measurement of intradiscal pressure (Wilke H-J et al., (1999) New in vivo measurements of pressures in the intervertebral disc in daily life, Spine 24:755-62). A ± 5 Nm torsional load and ± 1Nm flexion-extension, lateral bending load will be applied over 10 cycles whilst under a constant 0.5 MPa axial load. A cyclic axial load (0-1000N over 10 cycles) will be applied to investigate the axial compression response of the IVD.

### F. Pilot Studies

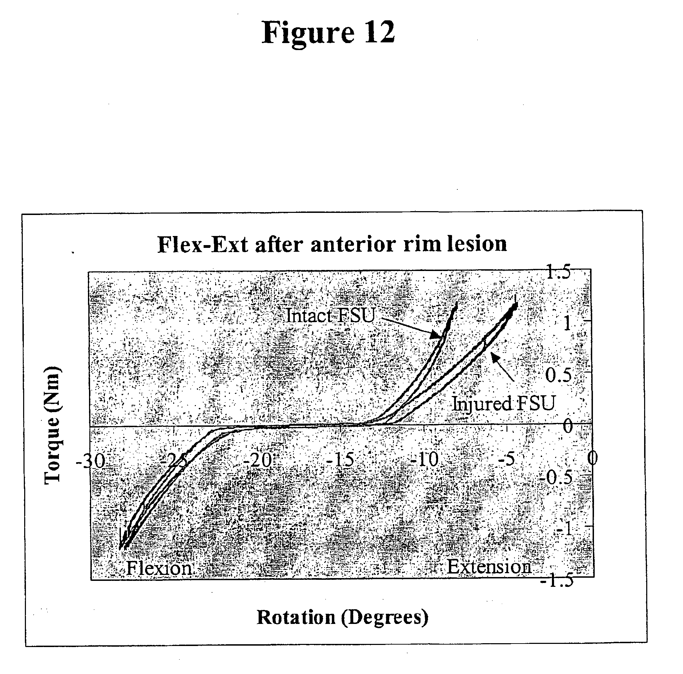

Pilot studies have been completed on both sheep and kangaroo spines to verify the experimental techniques. Figure 12 demonstrates typical 'Torque versus Rotation' plots of a sheep FSU over 10 flexion-extension loading cycles. The two plots represent the FSU before and after a circumferential anterior annular rim lesion. It can be seen that the annular cut resulted in increased range of motion (ROM) during extension, whilst flexion ROM was unaffected. This increased ROM overall represents an increase in spinal instability. Another observation is the high repeatability of the loading cycles, which verifies the reproducibility of the testing setup.

Data analysis will include stiffness in the linear region during the fifth loading cycle, hysteresis and strain energy and the extent of the neutral zone. Data from the non-operative levels will be compared with incised levels with and without OP-1 and a one-way repeated measures analysis of variance will be conducted on each of the biomechanical parameters.

### EXAMPLE 8: _The Effect Of OP-1 On Chondral And Microfracture Treated Cartilage Defects In A Goat Model

This study will evaluate the effects of OP-1 on the amount and composition of the reparative tissue induced by a microfracture procedure in a goat model. A total of 24 adult male goats (ages 1.5 to 3 years) weighing approximately 25 kg will be used. Prior to surgery, the knee joints will be roentgenographically examined to exclude animals with degenerative joint disease or other noted orthopedic problems. One 8mm (on a side) square chondral defect (cartilage removed down to tidemark-the calcified cartilage layer) will be produced in the trochlear groove of the right knees (stifle joints) of all animals. In 12 of the goats this chondral defect will serve as the site to the treated (Groups IA and IB (see table 4 below). The right knee joints of 12 of the animals will then undergo micro fracture treatment (Groups IIA and IIB). 16 microfracture holes will be produced using a pick of approximately 1 mm diameter.

Immediately postoperative, approximately 0.3 ml of OP-1 putty (collagen + CMC) hydrated with saline will be injected into the synovial fluid of the joint. At seven days, a second injection will be administered. In 6 of the animals in The chondral defect group (IB) and in 6 of the animals in the microfracture group (IIB) only vehicle will be delivered.

**Table 6**

| **Group** | **Type of Lesion** | **Treatment (+ or - OP-1)** | **Sample Size** |
|---|---|---|---|
| IA | Chondral | + | 6 |
| IB | Chondral | - | 6 |
| IIA | Microfracture | + | 6 |
| IIB | Microfracture | - | 6 |

All animals will be sacrificed 16 weeks after surgery. All of the sites will be prepared for histomorphometric evaluation. One histological section from the center portion of each defect will be evaluated. The total area and the percentages of specific tissue types (articular cartilage, hyaline cartilage, fibrocartilage and fibrous tissue) filling the original chondral defect region will be determined using a grid in the eyepiece of the microscope. Well accepted histological criteria for tissue types will be employed (see, e.g., Wang Q., et al. Healing of defects in canine articular cartilage: distribution of nonvascular alpha smooth muscle actin-containing cells, Wound Repair Regen. 8, pp. 145-158 (2000); Breinan HA, et al., Healing of canine articular cartilage defects treated with microfracture, a type II collagen matrix, or cultured autologous chondrocytes, J. Orthop. Res. 18, pp. 781-789 (2000); and Breinan, HA, et al., Effect of cultured autologous chondrocytes on repair of chondral defects in a canine model, J. Bone Joint Surg. 79A, pp. 1439-1451 (1997)).

The present disclosure relates to the following items:
1. A method of repairing a cartilage defect in a patient comprising the step of administering into the cartilage or into the area surrounding the cartilage a composition comprising a therapeutically effective amount of a morphogenic protein.
2. The method of item 1, wherein the cartilage is selected from the group consisting of articular cartilage and non-articular cartilage.
3. The method of item 2, wherein the non-articular cartilage is selected from the group consisting of a meniscus and an intervertebral disc.
4. The method of item 1, wherein the area surrounding the cartilage is synovial fluid.
5. The method of item 1, wherein the morphogenic protein is selected from the group consisting of OP-1, OP-2, OP-3, BMP-2, BMP-3, BMP-4, BMP-5, BMP-6, BMP-8, BMP-9, BMP-10, BMP-11, BMP-12, BMP-13, BMP-15, BMP-16, BMP-17, BMP-18, DPP, Vg1 Vgr, 60A protein, GDF-1, GDF-2, GDF-3, GDF-5, GDF-6, GDF-7, GDF-8, GDF-9, GDF-10, GDF-11, GDF-12, CDMP-1, CDMP-2, CDMP-3, NODAL, UNIVIN, SCREW, ADMP, NEURAL, and amino acid sequence variants thereof.
6. The method of item 1, wherein the morphogenic protein comprises an amino acid sequence having at least 70% homology with the C-terminal 102-106 amino acids, including the conserved seven cysteine domain, of human OP-1, said morphogenic protein being capable of inducing repair of the cartilage defect.
7. The method of item 1, wherein the morphogenic protein is selected from the group consisting of OP-1, BMP-5, BMP-6, GDF-5, GDF-6, GDF-7, CDMP-1, CDMP-2 and CDMP-3.
8. The method of item 7, wherein said morphogenic protein is OP-1.
9. The method of item 1, wherein the composition is selected from the group consisting of a gel, an aqueous solution, a paste and a putty.
10. The method of item 9, wherein the composition is formulated as a sustained release formulation or as a delayed clearance formulation.
11. The method of item 9, wherein the composition is an injectable formulation.
12. The method of item 9, wherein the composition is a gel.
13. The method of item 9, wherein the composition is an aqueous solution.
14. A method of regenerating or producing cartilage in a patient comprising the step of administering into the cartilage or the area surrounding the cartilage a composition comprising a therapeutically effective amount of a morphogenic protein.
15. The method of item 14, wherein the cartilage is selected from the group consisting of articular cartilage and non-articular cartilage.
16. The method of item 15, wherein the non-articular cartilage is selected from the group consisting of a meniscus and an intervertebral disc.
17. The method of item 14, wherein the area surrounding the cartilage is synovial fluid.
18. The method of item 14, wherein the morphogenic protein is selected from the group consisting of OP-1, OP-2, OP-3, BMP-2, BMP-3, BMP-4, BMP-5, BMP-6, BMP-8, BMP-9, BMP-10, BMP-11, BMP-12, BMP-13, BMP-15, BMP-16, BMP-17, BMP-18, DPP, Vg1, Vgr, 60A protein, GDF-1, GDF-2, GDF-3, GDF-5, GDF-6, GDF-7, GDF-8, GDF-9, GDF-10, GDF-11, GDF-12, CDMP-1, CDMP-2, CDMP-3, NODAL, UNIVIN, SCREW, ADMP, NEURAL, and amino acid sequence variants thereof.
19. The method of item 14, wherein the morphogenic protein comprises an amino acid sequence having at least 70% homology with the C-terminal 102-106 amino acids, including the conserved seven cysteine domain, of human OP-1, said morphogenic protein being capable of inducing repair of the cartilage defect.
20. The method of item 18, wherein the morphogenic protein is selected from the group consisting of OP-1, BMP-5, BMP-6, GDF-5, GDF-6, GDF-7, CDMP-1, CDMP-2 and CDMP-3.
21. The method of item 18, wherein said morphogenic protein is OP-1.
22. The method of item 14, wherein the composition is selected from the group consisting of a gel, an aqueous solution, a paste and a putty.
23. The method of item 22, wherein the composition is formulated as a sustained release formulation or as a delayed clearance formulation.
24. The method of item 22, wherein the composition is an injectable formulation.
25. The method of item 22, wherein the composition is a gel.
26. The method of item 22, wherein the composition is an aqueous solution.
27. A method of promoting cartilage growth or accelerating cartilage formation in a patient comprising the step of administering into the cartilage or into the area surrounding the cartilage a composition comprising a therapeutically effective amount of a morphogenic protein.
28. The method of item 27, wherein the cartilage is selected from the group consisting of articular cartilage and non-articular cartilage.
29. The method of item 28, wherein the non-articular cartilage is selected from the group consisting of a meniscus and an intervertebral disc.
30. The method of item 27, wherein the area surrounding the cartilage is synovial fluid.
31. The method of item 27, wherein the morphogenic protein is selected from the group consisting of OP-1, OP-2, OP-3, BMP-2, BMP-3, BMP-4, BMP-5, BMP-6, BMP-8, BMP-9, BMP-10, BMP-11, BMP-12, BMP-13, BMP-15, BMP-16, BMP-17, BMP-18, DPP, Vg1, Vgr, 60A protein, GDF-1, GDF-2, GDF-3, GDF-5, GDF-6, GDF-7, GDF-8, GDF-9, GDF-10, GDF-11, GDF-12, CDMP-1, CDMP-2, CDMP-3, NODAL, UNIVIN, SCREW, ADMP, NEURAL, and amino acid sequence variants thereof.
32. The method of item 27, wherein the morphogenic protein comprises an amino acid sequence having at least 70% homology with the C-terminal 102-106 amino acids, including the conserved seven cysteine domain, of human OP-1, said morphogenic protein being capable of inducing repair of the cartilage defect.
33. The method of item 31, wherein the morphogenic protein is selected from the group consisting of OP-1, BMP-5, BMP-6, GDF-5, GDF-6, GDF-7, CDMP-1, CDMP-2 and CDMP-3.
34. The method of item 33, wherein said morphogenic protein is OP-1.
35. The method of item 27, wherein the composition is selected from the group consisting of a gel, an aqueous solution, a paste and a putty.
36. The method of item 35, wherein the composition is formulated as a sustained release formulation or as a delayed clearance formulation.
37. The method of item 35, wherein the composition is an injectable formulation.
38. The method of item 35, wherein the composition is a gel.
39. The method of item 35, wherein the composition is an aqueous solution.
40. A method of preventing cartilage degradation or treating cartilage injury or degenerative disease or disorder in a patient comprising the step of administering into the cartilage or into the area surrounding the cartilage a composition comprising a therapeutically effective amount of a morphogenic protein.
41. The method of item 40, wherein the cartilage is selected from the group consisting of articular cartilage and non-articular cartilage.
42. The method of item 31, wherein the non-articular cartilage is selected from the group consisting of a meniscus and an intervertebral disc.
43. The method of item 40, wherein the area surrounding the cartilage is synovial fluid.
44. The method of item 40, wherein the morphogenic protein is selected from the group consisting of OP-1, OP-2, OP-3, BMP-2, BMP-3, BMP-4, BMP-5, BMP-6, BMP-8, BMP-9, BMP-10, BMP-11, BMP-12, BMP-13, BMP-15, BMP-16, BMP-17. BMP-18, DPP, Vg1, Vgr, 60A protein, GDF-1, GDF-2, GDF-3, GDF-5, GDF-6, GDF-7, GDF-8, GDF-9, GDF-10, GDF-11, GDF-12, CDMP-1, CDMP-2, CDMP-3, NODAL, UNIVIN, SCREW, ADMP, NEURAL, and amino acid sequence variants thereof.
45. The method of item 40, wherein the morphogenic protein comprises an amino acid sequence having at least 70% homology with the C-terminal 102-106 amino acids, including the conserved seven cysteine domain, of human OP-1, said morphogenic protein being capable of inducing repair of the cartilage defect.
46. The method of item 45, wherein the morphogenic protein is selected from the group consisting of OP-1, GDF-5, GDF-6, GDF-7, CDMP-1, CDMP-2 and CDMP-3.
47. The method of item 46, wherein said morphogenic protein is OP-1.
48. The method of item 40, wherein the composition is selected from the group consisting of a gel, an aqueous solution, a paste and a putty.
49. The method of item 48, wherein the composition is formulated as a sustained release formulation or as a delayed clearance formulation.
50. The method of item 48, wherein the composition is an injectable formulation.
51. The method of item 48, wherein the composition is a gel.
52. The method of item 48, wherein the composition is an aqueous solution.
53. The method of item 40, wherein the tissue injury or degenerative disease is selected from the group consisting of osteoarthritis, meniscus tears, ACL injury and disc degeneration.

## Claims

1. A pharmaceutical composition consisting of OP-1 and a pharmaceutically acceptable carrier for use in repairing an articular cartilage defect in a patient wherein the cartilage is surrounded by synovial fluid; wherein the pharmaceutically acceptable carrier is selected from water, saline, Ringer's solution, a buffered solution, hyaluronan and dextrose solution; wherein the pharmaceutical composition is a liquid solution or liquid suspension; and wherein the pharmaceutical composition is to be administered into the synovial fluid surrounding the articular cartilage.

2. A pharmaceutical composition consisting of OP-1 and a pharmaceutically acceptable carrier for use in regenerating or producing articular cartilage at a cartilage defect site in a patient wherein the articular cartilage is surrounded by synovial fluid; wherein the pharmaceutically acceptable carrier is selected from water, saline, Ringer's solution, a buffered solution, hyaluronan and dextrose solution; wherein the pharmaceutical composition is a liquid solution or liquid suspension; and wherein the pharmaceutical composition is to be administered into the synovial fluid surrounding the articular cartilage.

3. A pharmaceutical composition consisting of OP-1 and a pharmaceutically acceptable carrier for use in promoting articular cartilage growth or accelerating articular cartilage formation at a cartilage defect site in a patient wherein the articular cartilage is surrounded by synovial fluid; wherein the pharmaceutically acceptable carrier is selected from water, saline, Ringer's solution, a buffered solution, hyaluronan and dextrose solution; wherein the pharmaceutical composition is a liquid solution or liquid suspension; and wherein the pharmaceutical composition is to be administered into the synovial fluid surrounding the articular cartilage.

4. A pharmaceutical composition consisting of OP-1 and a pharmaceutically acceptable carrier for use in preventing articular cartilage degradation or treating articular cartilage injury or degenerative disease or disorder in a patient wherein the articular cartilage is surrounded by synovial fluid; wherein the pharmaceutically acceptable carrier is selected from water, saline, Ringer's solution, a buffered solution, hyaluronan and dextrose solution; wherein the pharmaceutical composition is a liquid solution or liquid suspension; and wherein the pharmaceutical composition is to be administered into the synovial fluid surrounding the articular cartilage.

5. The pharmaceutical composition for use according to any one of claims 1-4, wherein the composition is an injectable formulation.

6. The pharmaceutical composition for use according to any one of claims 1-5, wherein the composition is an aqueous solution.

7. The pharmaceutical composition for use according to any one of claims 1-6, wherein the OP-1 is glycosylated.

8. The pharmaceutical composition for use according to any one of claims 4-7, wherein the articular cartilage injury or degenerative disease is selected from the group consisting of osteoarthritis, and ACL injury.

## Patentansprüche

1. Pharmazeutische Zusammensetzung bestehend aus OP-1 und einem pharmazeutisch verträglichen Träger zur Verwendung in der Reparatur eines Gelenkknorpelschadens in einem Patienten, wobei der Knorpel von Gelenkschmiere umgeben ist; wobei der pharmazeutisch verträgliche Träger ausgewählt ist aus Wasser, Kochsalzlösung, Ringer-Lösung, einer Pufferlösung, Hyaluronsäure- und Dextrose-Lösung; wobei die pharmazeutische Zusammensetzung eine flüssige Lösung oder eine flüssige Suspension ist; und wobei die pharmazeutische Zusammensetzung in die den Gelenkknorpel umgebende Gelenkschmiere zu verabreichen ist.

2. Pharmazeutische Zusammensetzung bestehend aus OP-1 und einem pharmazeutisch verträglichen Träger zur Verwendung in der Regeneration oder Produktion von Gelenkknorpel an einer Stelle mit geschädigtem Knorpel in einem Patienten, wobei der Gelenkknorpel von Gelenkschmiere umgeben ist; wobei der pharmazeutisch verträgliche Träger ausgewählt ist aus Wasser, Kochsalzlösung, Ringer-Lösung, einer Pufferlösung, Hyaluronsäure- und Dextrose-Lösung; wobei die pharmazeutische Zusammensetzung eine flüssige Lösung oder eine flüssige Suspension ist; und wobei die pharmazeutische Zusammensetzung in die den Gelenkknorpel umgebende Gelenkschmiere zu verabreichen ist.

3. Pharmazeutische Zusammensetzung bestehend aus OP-1 und einem pharmazeutisch verträglichen Träger zur Verwendung bei Förderung von Gelenkknorpelwachstum oder zur Beschleunigung der Gelenkknorpelbildung an einer Stelle mit geschädigtem Knorpel in einem Patienten, wobei der Gelenkknorpel von Gelenkschmiere umgeben ist; wobei der pharmazeutisch verträgliche Träger ausgewählt ist aus Wasser, Kochsalzlösung, Ringer-Lösung, einer Pufferlösung, Hyaluronsäure- und Dextrose-Lösung; wobei die pharmazeutische Zusammensetzung eine flüssige Lösung oder eine flüssige Suspension ist; und wobei die pharmazeutische Zusammensetzung in die den Gelenkknorpel umgebende Gelenkschmiere zu verabreichen ist.

4. Pharmazeutische Zusammensetzung bestehend aus OP-1 und einem pharmazeutisch verträglichen Träger zur Vorbeugung von Gelenkknorpelabbau oder zur Behandlung einer Gelenkknorpelverletzung oder einer degenerativen Erkrankung oder Störung bei einem Patienten, wobei der Gelenkknorpel von Gelenkschmiere umgeben ist; wobei der pharmazeutisch verträgliche Träger ausgewählt ist aus Wasser, Kochsalzlösung, Ringer-Lösung, einer Pufferlösung, Hyaluronsäure- und Dextrose-Lösung; wobei die pharmazeutische Zusammensetzung eine flüssige Lösung oder eine flüssige Suspension ist; und wobei die pharmazeutische Zusammensetzung in die den Gelenkknorpel umgebende Gelenkschmiere zu verabreichen ist.

5. Pharmazeutische Zusammensetzung zur Verwendung nach einem der Ansprüche 1 bis 4, wobei die Zusammensetzung eine injizierbare Formulierung ist.

6. Pharmazeutische Zusammensetzung zur Verwendung nach einem der Ansprüche 1 bis 5, wobei die Zusammensetzung eine wässrige Lösung ist.

7. Pharmazeutische Zusammensetzung zur Verwendung nach einem der Ansprüche 1 bis 6, wobei OP-1 glycosyliert ist.

8. Pharmazeutische Zusammensetzung zur Verwendung nach einem der Ansprüche 4 bis 7, wobei die Gelenkknorpelverletzung oder degenerative Erkrankung ausgewählt ist aus der Gruppe bestehend aus Osteoarthritis und Verletzung des vorderen Kreuzbandes (ACL).

## Revendications

1. Composition pharmaceutique consistant en OP-1 et un porteur acceptable pharmaceutiquement pour utilisation dans la réparation d'un défaut de cartilage articulaire dans un patient, où le cartilage est entouré par le fluide synovial ; où le porteur acceptable pharmaceutiquement est choisi à partir de l'eau, d'une solution saline, d'une solution de Ringer, d'une solution tamponnée, d'une solution d'acide hyaluronique et de dextrose ; où la composition pharmaceutique est une solution liquide ou une suspension liquide ; et où la composition pharmaceutique doit être administrée dans le fluide synovial entourant le cartilage articulaire.

2. Composition pharmaceutique consistant en OP-1 et un porteur acceptable pharmaceutiquement pour utilisation dans la régénération ou la production de cartilage articulaire à un site de défaut de cartilage dans un patient où le cartilage articulaire est entouré par du fluide synovial ; où le porteur acceptable pharmaceutiquement est choisi à partir de l'eau, d'une solution saline, d'une solution de Ringer, d'une solution tamponnée, d'une solution d'acide hyaluronique et de dextrose ; où la composition pharmaceutique est une solution liquide ou une suspension liquide ; et où la composition pharmaceutique doit être administrée dans le fluide synovial entourant le cartilage articulaire.

3. Composition pharmaceutique consistant en OP-1 et un porteur acceptable pharmaceutiquement pour utilisation dans la favorisation de croissance du cartilage articulaire ou l'accélération de la formation de cartilage articulaire à un site de défaut de cartilage à un patient où le cartilage articulaire est entouré par du fluide synovial ; où le porteur acceptable pharmaceutiquement est choisi à partir de l'eau, d'une solution saline, d'une solution de Ringer, d'une solution tamponnée, d'une solution d'acide hyaluronique et de dextrose ; où la composition pharmaceutique est une solution liquide ou une suspension liquide ; et où la composition pharmaceutique doit être administrée dans le fluide synovial entourant le cartilage articulaire.

4. Composition pharmaceutique consistant en OP-1 et un porteur acceptable pharmaceutiquement pour utilisation dans la prévention de dégradation de cartilage articulaire ou le traitement de dommages de cartilage articulaire ou d'une infection dégénérative ou le trouble dans un patient où le cartilage articulaire est entouré par du fluide synovial ; où le porteur acceptable pharmaceutiquement est choisi à partir de l'eau, d'une solution saline, d'une solution de Ringer, d'une solution tamponnée, d'une solution d'acide hyaluronique et de dextrose ; où la composition pharmaceutique est une solution liquide ou une suspension liquide; et où la composition pharmaceutique doit être administrée dans le fluide synovial entourant le cartilage articulaire.

5. La composition pharmaceutique pour utilisation selon l'une quelconque des revendications 1-4, où la composition est une.formulation injectable.

6. La composition pharmaceutique pour utilisation selon l'une quelconque des revendications 1-5, où la composition est une solution aqueuse.

7. La composition pharmaceutique pour utilisation selon l'une quelconque des revendications 1-6, où le OP-1 est glycosylé.

8. La composition pharmaceutique pour utilisation selon l'une quelconque des revendications 4-7, où le dommage de cartilage articulaire ou l'affection dégénérative est choisi dans le groupe consistant en ostéoarthrite et dommage ACL.
